# EUROPÄISCHE PATENTSCHRIFT

(11) **EP 1 501 897 B1**
(45) Veröffentlichungstag und Bekanntmachung des Hinweises auf die Patenterteilung: **14.03.2012**
(21) Anmeldenummer: 03717301.0
(22) Anmeldetag: 15.04.2003
(51) Int. Cl.: C09B 5/62, C09B 57/08, C07D 221/14

(54) **VERFAHREN ZUR HERSTELLUNG VON PERYLEN-3,4:9,10-TETRACARBONSÄUREDIIMIDEN UND PERYLEN-3,4:9,10-TETRACARBONSÄUREDIANHYDRID SOWIE VON NAPHTHALIN-1,8-DICARBONSÄUREIMIDEN**
METHOD FOR THE PRODUCTION OF PERYLEN-3,4:9,10-TETRACARBOXYLIC ACID DIIMIDES AND PERYLEN-3,4:9,10-TETRACARBOXYLIC ACID DIANHYDRIDE AND NAPHTALENE-1,8-DICARBOXYLIMIDES
PROCEDE POUR LA PRODUCTION DE DIIMIDES D'ACIDE PERYLENE-3,4:9,10-TETRACARBOXYLIQUE ET DE DIANHYDRIDE D'ACIDE PERYLENE-3,4:9,10-TETRACARBOXYLIQUE AINSI QUE D'IMIDES D'ACIDE NAPHTALENE-1,8-DICARBOXYLIQUE

(30) Priorität: 25.04.2002 DE 10218618
(43) Veröffentlichungstag der Anmeldung: 02.02.2005
(73) Patentinhaber: BASF SE, 67056 Ludwigshafen (DE)
(72) Erfinder: DUNG, Bernhard, 68169 Mannheim (DE); MÜLLER, Felix, 67227 Frankenthal (DE); BÖHM, Arno, 68305 Mannheim (DE); HELFER, Willi, 67159 Friedelsheim (DE); WEYRAUCH, Volker, 67146 Deidesheim (DE); HENNING, Georg, 67061 Ludwigshafen (DE)
(86) Internationale Anmeldenummer: PCT/EP2003/003901
(87) Internationale Veröffentlichungsnummer: WO 2003/091345

(56) Entgegenhaltungen:
- EP-A- 0 525 538
- US-A- 4 197 111
- US-A- 4 919 848
- CHEMICAL ABSTRACTS, vol. 117, no. 6, 10. August 1992 (1992-08-10) Columbus, Ohio, US; abstract no. 50766j, H.OKAZAKI ET AL.: "Manufacture of perylene-3,4,9,10-tetracarboxylic acid diimide" Seite 108; XP002251037 -& JP 03 223282 A (NIPPON STEEL CHEMICAL CO., LTD.) 2. Oktober 1991 (1991-10-02)
- CHEMICAL ABSTRACTS, vol. 86, no. 12, 21. März 1977 (1977-03-21) Columbus, Ohio, US; abstract no. 74420j, Z.JANKOWSKI ET AL: "N-(1-benzotriazolyl)naphthalimide derivatives" Seite 93; XP002251038 & PL 78 458 A 14. Juli 1975 (1975-07-14)

## Beschreibung

Die vorliegende Erfindung betrifft ein neues Verfahren zur Herstellung von Perylen-3,4:9,10-tetracarbonsäurediimiden der allgemeinen Formel I 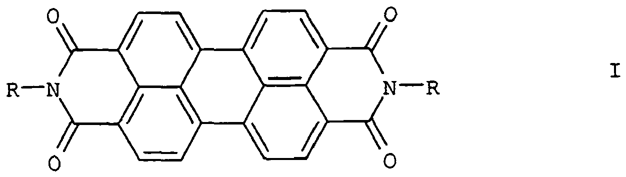 in der die Variablen folgende Bedeutung haben:
R C₁-C₃₀-Alkyl, dessen Kohlenstoffkette durch eine oder mehrere Gruppierungen -O- unterbrochen und/oder das durch C₅-C₈-Cycloalkyl, das durch C₁-C₆-Alkyl ein- oder mehrfach substituiert sein kann, Phenyl oder Phenyl-C₁-C₆-alkyl, das jeweils durch C₁-C₁₈-Alkyl und/oder C₁-C₆-Alkoxy ein- oder mehrfach substituiert sein kann, -OCOR¹, -N(R¹)₂, -SO₂NH₂, -SO₂N(R¹)₂, -CON(R¹)₂ und/oder -COOR¹ ein- oder mehrfach substituiert sein kann;
   C₅-C₈-Cycloalkyl, dessen Kohlenstoffgerüst durch eine oder mehrere Gruppierungen -O-, -S- und/oder -NR²- unterbrochen sein kann und/oder das durch C₁-C₆-Alkyl ein- oder mehrfach substituiert sein kann;
   Phenyl, Phenyl-C₁-C₆-alkyl, Naphthyl oder Hetaryl, das jeweils durch C₁-C₁₈-Alkyl, C₁-C₆-Alkoxy, Phenylazo, Naphthylazo, Pyridylazo, Pyrimidylazo, Cyano, -N(R¹)₂, -CON(R¹)₂ und/ oder -COOR¹ ein- oder mehrfach substituiert sein kann;
R¹ C₁-C₆-Alkyl, C₅-C₈-Cycloalkyl, Phenyl oder Phenyl-C₁-C₆-alkyl;
R² C₁-C₆-Alkyl, Phenyl oder Phenyl-C₁-C₆-alkyl,
durch Dimerisierung eines Naphthalin-1,8-dicarbonsäureimids der Formel II

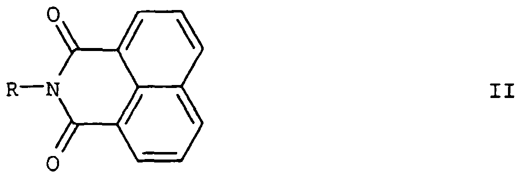

Außerdem betrifft die Erfindung die Herstellung von Perylen-3,4:9,10-tetracarbonsäuredianhydrid durch Modifizierung des Verfahrens zur Herstellung der Perylen-3,4:9,10-tetracarbonsäurediimide.

Weiterhin betrifft die Erfindung die Herstellung der als Ausgangsprodukt für diese Herstellungsverfahren dienenden Naphthalin-1,8-dicarbonsäureimide II sowie neue Naphthalin-1,8-dicarbonsäureimide IIb.

Perylen-3,4:9,10-tetracarbonsäurediimide (I; im folgenden kurz "Perylimide" genannt) finden seit langem aufgrund ihrer herausragenden anwendungstechnischen Eigenschaften (Farbbrillanz, hohe thermische, chemische und photochemische Stabilität, hohe Fluoreszenz) als Küpenfarbstoffe, Pigmente und Fluoreszenzfarbstoffe Verwendung. Weiterhin werden die Perylimide I für reprographische Prozesse, in der Elektrophotographie, in Fluoreszenzsolarkollektoren, in der Photovoltaik, als Laserfarbstoffe, als Aktivkomponente in Chemilumineszenzanwendungen, in elektrolumineszenten Vorrichtungen und in Modellsystemen für molekulare Schalter eingesetzt.

Es ist seit langem bekannt, daß unsubstituiertes Perylimid durch Verschmelzen von Naphthalin-1,8-dicarbonsäureimid (im folgenden kurz "Naphthalimid" genannt) mit Kaliumhydroxid oder Mischungen von Kaliumhydroxid und Natriumhydroxid und anschließende Reoxidation der intermediär gebildeten "Leukoform" hergestellt werden kann (vgl. z.B. EP-A-525 538). Bei dieser Kondensation können Natriumacetat (EP-A-54 806) oder Glykole (US-A-3 446 810) zur Erhöhung der Ausbeute zugesetzt werden.

Analog sind, allerdings in unbefriedigender Ausbeute und nur unter erheblichem apparativen Aufwand zur Abtrennung unerwünschter Verunreinigungen, durch alkalisches Verschmelzen von N-Methyl-, N-Ethyl- und N-Phenylnaphthalimid die entsprechenden N,N'-disubstituierten Perylimide zu erhalten (DE-PS-276 956). Weitere N,N'-disubstituierte Perylimide, z.B. solche, die an den Stickstoffatomen durch substituierte Phenylreste oder durch funktionalisierte Alkylreste substituiert sind, können nach diesem Verfahren nicht hergestellt werden. Zudem ist die aus ökologischen und ökonomischen Gründen notwendige Wiederaufarbeitung der verwendeten Schmelzen sehr energieintensiv und technisch aufwendig.

In Kawamura Rikagaku Kenkyusho Hokoku 8, Seite 85-95 (1996, veröffentlicht 1997) (Chemical Abstracts 127: 264199g) ist die Herstellung von unsubstituiertem Perylimid durch Dimerisierung von Naphthalimid in Diethylenglykoldimethylether in Gegenwart von Kalium-tert.-butylat und 1,5-Diazabicyclo-[4.3.0]-5-nonen beschrieben. Nach dem hier vorgestellten Reaktionsmechanismus ist die gleichzeitige Anwesenheit der sterisch gehinderten bicyclischen Stickstoffbase, der Kalium enthaltenden Base und des chelatisierenden Lösungsmittels essentiell und führt zur Bildung eines Komplexes, welcher die Bildung des Perylimids ermöglicht.

In der zugehörigen JP-A-194 746/1997 sowie J. Org. Chem. 2001, 66, Seite 94-98 werden auch Heptan, Toluol, Chinolin und Cyclohexylamin als Lösungsmittel für die Herstellung von unsubstituiertem Perylimid eingesetzt. Weiterhin sollen in Diethylenglykoldimethylether auch N,N'-Dimethyl-, N,N'-Dioctyl-, N,N'-Dicyclohexyl-, N,N'-Diphenyl-, N,N'-Bis(p-methoxyphenyl)-, N,N'-Bis(p-chlorphenyl)-, N,N'-Bis(3,5-dimethylphenyl)-, N,N'-Dibenzyl- und N,N'-Bis(p-phenylazophenyl)perylimid erhältlich sein. Die Ausbeuten für diese Produkte schwanken und liegen bei der überwiegenden Zahl der Substrate selbst bei Verwendung extremer Basenüberschüsse (27 Äquivalente Kalium-tert.-butylat und 27 Äquivalente 1,5-Diazabicyclo-[4.3.0]-5-nonen) nur bei < 50%.

Dieses Verfahren hat neben den niedrigen Ausbeuten eine Reihe von weiteren Nachteilen. So läßt die Reinheit der erhaltenen substituierten Perylimide oft zu wünschen übrig. Zudem ist das Verfahren aufgrund des hohen Preises der in großem Überschuß eingesetzten bicyclischen Stickstoffbasen auch bei deren weitgehender, nur über eine aufwendige fraktionierte Destillation zu bewerkstelligender Rückführung aus Kostengründen nicht im industriellen Maßstab einsetzbar.

Zur Herstellung der als Ausgangsprodukte für die Perylimidherstellung dienenden Naphthalimide II sind verschiedene von Naphthalin-1,8-dicarbonsäureanhydrid ausgehende Verfahren beschrieben: die Umsetzung mit Anilin in Essigsäure, mit tert.-Butylamin, 1,1-Dimethylpropylamin oder o-Nitroanilin in Substanz, mit primären C₁-C₄-Alkylaminen in Benzol, Toluol, Chlorbenzol, Pyridin oder Picolin in Anwesenheit von Phosphortrichlorid, Phosphorylchlorid oder Thionylchlorid sowie mit kurzkettigen, wasserlöslichen Alkylaminen in wäßrigen Medien (vgl. Pol. J. Chem. 55, Seite 555-563 (1981), Zh. Org. Khim. 21, Seite 2415-2423 (1985), IN 141431).

JP 03223282 A2 offenbart ein Verfahren zur Herstellung von Stickstoff-unsubstituierten Perylen-3,4,9,10-tetracarbonsäurediimiden durch Dimerisierung von Naphthalin-1,8-dicarbonsäureimid in Gegenwart eines Alkalimetallhydroxids. Ein Verfahren zur Herstellung von N,N'-substituierter Perylen-3,4,9,10-tetracarbonsäurediimide wird nicht offenbart.

US 4,919,848 offenbart ein Verfahren zur Herstellung N-substituierten Naphthalimiden durch Umsetzung von Naphthalin-1,8-dicarbonsäure mit organischen Aminen in Gegenwart von Toluol und Essigsäure unter azeotroper Abdestillation von Wasser.

US 4,197,111 offenbart Verfahren zur Herstellung N-substituierter Naphthalimide durch Umsetzung von Naphthalin-1,8-dicarbonsäureanhydrid mit organischen Aminen in Toluol oder durch Umsetzung von N-Amino-1,8-naphtalimiden mit Isocyanaten.

PL 78458, auch veröffentlicht in Chemical Abstracts, Bd 86, Nr. 12, No. 74420j, offenbart Fluoreszenzfarbstoffe mit Benzotriazolylgruppen durch Acylierung von Naphthalin-1,8-dicarbonsäureanhydrid mit 2-Nitrophenylhydrazin oder Derivaten hiervon.

Diese Verfahren sind jedoch in verschiedener Hinsicht nicht zufriedenstellend: Es werden nur unbefriedigende Ausbeuten erzielt und/oder verunreinigte Produkte erhalten. Außerdem sind die Verfahren nicht universell einsetzbar. Nur wenige primäre Amine können so mit Naphthalin-1,8-dicarbonsäureanhydrid umgesetzt werden, insbesondere sterisch stark gehinderte oder reaktionsträge aromatische Amine können nicht eingesetzt werden.

Der Erfindung lag daher die Aufgabe zugrunde, den genannten Mängeln abzuhelfen und die Perylimide I und die Naphthalimide II als Vorprodukte der Perylimide I auf vorteilhafte Weise zugänglich zu machen.

Demgemäß wurde ein Verfahren zur Herstellung von Perylen-3,4:9,10-tetracarbonsäurediimiden der allgemeinen Formel I 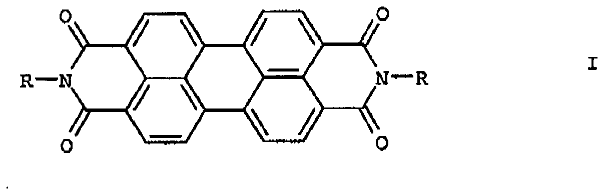 in der die Variablen folgende Bedeutung haben:
R C₁-C₃₀-Alkyl, dessen Kohlenstoffkette durch eine oder mehrere Gruppierungen -0- unterbrochen und/oder das durch C₅-C₈-Cycloalkyl, das durch C₁-C₆-Alkyl ein- oder mehrfach substituiert sein kann, Phenyl oder Phenyl-C₁-C₆-alkyl, das jeweils durch C₁-C₁₈-Alkyl und/oder C₁-C₆-Alkoxy ein- oder mehrfach substituiert sein kann, -OCOR¹, -N(R¹)₂, -SO₂NH₂, -SO₂N(R¹)₂. -CON(R¹)₂ und/oder -COOR¹ ein- oder mehrfach substituiert sein kann;
   C₅-C₈-Cycloalkyl, dessen Kohlenstoffgerüst durch eine oder mehrere Gruppierungen -O-, -S- und/oder -NR²- unterbrochen sein kann und/oder das durch C₁-C₆-Alkyl ein- oder mehrfach substituiert sein kann; Phenyl, Phenyl-C₁-C₆-alkyl, Naphthyl oder Hetaryl, das jeweils durch C₁-C₁₈-Alkyl, C₁-C₆-Alkoxy, Phenylazo, Naphthylazo, Pyridylazo, Pyrimidylazo, Cyano, -N(R¹)₂, -CON(R¹)₂ und/ oder -COOR¹ ein- oder mehrfach substituiert sein kann;
R¹ C₁-C₆-Alkyl, C₅-C₈-Cycloalkyl, Phenyl oder Phenyl-C₁-C₆-alkyl;
R² C₁-C₆-Alkyl, Phenyl oder Phenyl-C₁-C₆-alkyl,
durch Dimerisierung eines Naphthalin-1,8-dicarbonsäureimids der Formel II 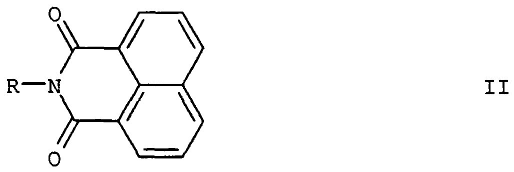 gefunden, welches dadurch gekennzeichnet ist, daß man die Dimerisierung in einem im wesentlichen aus einem unpolar-aprotischen organischen Lösungsmittel und einer alkalimetallhaltigen Base bestehenden Reaktionsmedium durchführt und die dabei als Alkalimetallsalz anfallende Leukoform des Perylen-3,4:9,10-tetracarbon-säurediimids anschließend in Gegenwart eines polaren Lösungsmittels reoxidiert.

Weiterhin wurde ein Verfahren zur Herstellung von Perylen-3,4:9,10-tetracarbonsäuredianhydrid gefunden, welches dadurch gekennzeichnet ist, daß man ein Naphthalin-1,8-dicarbon-säureimid der Formel IIa 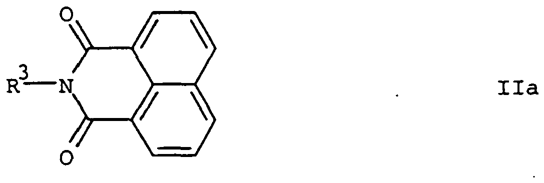 in der R³ Cyclohexyl oder Phenyl, das jeweils durch bis zu drei C₁-C₄-Alkylreste substituiert sein kann, bedeutet, in einem im wesentlichen aus einem unpolar-aprotischen organischen Lösungsmittel und einer alkalimetallhaltigen Base bestehenden Reaktionsmedium dimerisiert und die anschließende Reoxidation der dabei als Alkalimetallsalz anfallenden Leukoform des Perylen-3,4:9,10-tetracarbonsäurediimids der Formel Ia 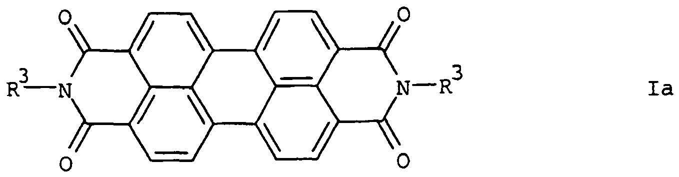 in Gegenwart eines inerten organischen Lösungsmittels, einer alkalimetallhaltigen Base und von Wasser vornimmt, so daß das Diimid zum Tetraalkalimetallsalz der Perylen-3,4:9,10-tetracarbonsäure verseift wird, und abschließend dieses Salz unter Einwirkung einer wäßrigen anorganischen Säure in das Perylen-3,4:9,10-tetracarbonsäuredianhydrid überführt.

Außerdem wurde ein Verfahren zur Herstellung von Naphthalin-1,8-dicarbonsäureimiden der allgemeinen Formel II 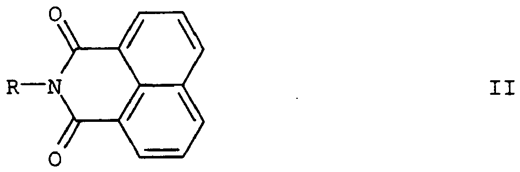 durch Umsetzung von Naphthalin-1,8-dicarbonsäureanhydrid mit einem primären Amin der allgemeinen Formel III

R-NH₂ III

gefunden, welches dadurch gekennzeichnet ist, daß man die Umsetzung in Gegenwart eines polar-aprotischen organischen Lösungsmittels sowie einer organischen oder anorganischen Säure oder eines sauren Übergangsmetallsalzes als Katalysator-oder in Gegenwart von Phenol vornimmt.

Nicht zuletzt wurden die Naphthalin-1,8-dicarbonsäureimide der allgemeinen Formel IIb 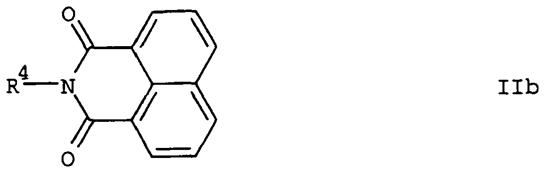 gefunden, in welcher die Variablen folgende Bedeutung haben:
R⁴ C₁-C₃₀-Alkyl, das durch C₅-C₈-Cycloalkyl, das durch C₁-C₆-Alkyl ein- oder mehrfach substituiert sein kann, Phenyl oder Phenyl-C₁-C₆-alkyl, das jeweils durch C₁-C₁₈-Alkyl und/oder C₁-C₆-Alkoxy ein- oder mehrfach substituiert sein kann, -OCOR¹, -N(R¹)₂, -SO₂NH₂, -SO₂N(R¹)₂, -CON(R¹)₂ und/oder -COOR¹ ein- oder mehrfach substituiert ist und dessen Kohlenstoffkette durch eine oder mehrere Gruppierungen -O- unterbrochen sein kann;
   C₅-C₈-Cycloalkyl, dessen Kohlenstoffgerüst durch eine oder mehrere Gruppierungen -O-, -S- und/oder -NR²- unterbrochen und/oder das durch C₁-C₆-Alkyl ein- oder mehrfach substituiert ist;
   Phenyl oder Phenyl-C₁-C₆-alkyl, das jeweils durch C₁-C₁₈-Alkyl, C₁-C₆-Alkoxy, Phenylazo, Naphthylazo, Pyridylazo, Pyrimidylazo, Cyano, -N(R¹)₂, -CON(R¹)₂ und/ oder -COOR¹ ein- oder mehrfach substituiert ist;
   Naphthyl oder Hetaryl, das jeweils durch C₁-C₁₈-Alkyl, C₁-C₆-Alkoxy, Phenylazo, Naphthylazo, Pyridylazo, Pyrimidylazo, Cyano, -N(R¹)₂, -CON(R¹)₂ und/ oder -CO0R¹ ein- oder mehrfach substituiert sein kann;
R¹ C₁-C₆-Alkyl, C₅-C₈-Cycloalkyl, Phenyl oder Phenyl-C₁-C₆-alkyl;
R² C₁-C₆-Alkyl, Phenyl oder Phenyl-C₁-C₆-alkyl.

Alle in den Formeln I bis III auftretenden Alkylgruppen können sowohl geradkettig als auch verzweigt sein. Aromatische Reste, die substituiert sind, können im allgemeinen bis zu 3, bevorzugt 1 oder 2, der genannten Substituenten tragen.

Als Beispiel für geeignete R, R¹, R², R³ und R⁴ (sowie deren Substituenten) seien im einzelnen genannt:
Methyl, Ethyl, Propyl, Isopropyl, Butyl, Isobutyl, tert.-Butyl, Pentyl, Isopentyl, Neopentyl, tert.-Pentyl, Hexyl, 2-Methylpentyl, Heptyl, 1-Ethylpentyl, Octyl, 2-Ethylhexyl, Isooctyl, Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl und Eicosyl (die obigen Bezeichnungen Isooctyl, Isononyl, Isodecyl und Isotridecyl sind Trivialbezeichnungen und stammen von den nach der Oxosynthese erhaltenen Alkoholen);
Methoxy, Ethoxy, Propoxy, Isopropoxy, Butoxy, Isobutoxy, sec.-Butoxy, tert.-Butoxy, Pentoxy, Isopentoxy, Neopentoxy, tert.-Pentoxy und Hexoxy;
N,N-Dimethylamino, N,N-Diethylamino, N,N-Dipropylamino, N,N-Diisopropylamino, N,N-Dibutylamino, N,N-Diisobutylamino, N,N-Dipentylamino, N,N-Dihexylamino, N,N-Dicyclopentylamino, N,N-Dicyclohexylamino, N,N-Dicycloheptylamino, N,N-Diphenylamino und N,N-Dibenzylamino;
Methoxymethyl, 2-Methoxyethyl, 2-Ethoxyethyl, 2-Propoxyethyl, 2-Isopropoxyethyl, 2-Butoxyethyl, 2- und 3-Methoxypropyl, 2- und 3-Ethoxypropyl, 2- und 3-Propoxypropyl, 2- und 3-Butoxypropyl, 2-und 4-Methoxybutyl, 2- und 4-Ethoxybutyl, 2- und 4-Propoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 2- und 4-Butoxybutyl, 4,8-Dioxadecyl, 3,6,9-Trioxadecyl, 3,6,9-Trioxaundecyl, 3,6,9-Trioxadodecyl, 3,6,9,12-Tetraoxatridecyl und 3,6,9,12-Tetraoxatetradecyl;
2-Formyloxyethyl, 2- und 3-Formyloxypropyl, 2-, 3- und 4-Formyloxybutyl, 2-, 3-, 4-, 5- und 6-Formyloxyhexyl, 2-, 3-, 4-, 5-, 6-, 7- und 8-Formyloxyoctyl, 2-Acetoxyethyl, 2- und 3-Acetoxypropyl, 2-, 3- und 4-Acetoxybutyl, 2-, 3-, 4-, 5- und 6-Acetoxyhexyl, 2-, 3-, 4-, 5-, 6-, 7- und 8-Acetoxyoctyl, 2-Propionyloxyethyl, 2- und 3-Propionyloxypropyl, 2-, 3- und 4-Propionyloxybutyl, 2-, 3-, 4-, 5- und 6-Propionyloxyhexyl, 2-, 3-, 4-, 5-, 6-, 7- und 8-Propionyloxyoctyl, 2-Benzoyloxyethyl, 2- und 3-Benzoyloxypropyl, 2-, 3- und 4-Benzoyloxybutyl, 2-, 3-, 4-, 5-und 6-Benzoyloxyhexyl und 2-, 3-, 4-, 5-, 6-, 7- und 8-Benzoyloxyoctyl;
2-(N,N-Dimethyl-, N,N-Diethyl-, N,N-Dibutyl- und N,N-Diphenylamino)ethyl, 3-(N,N-Dimethyl-, N,N-Diethyl-, N,N-Dibutyl- und N,N-Diphenylamino)propyl, 4-(N,N-Dimethyl-, N,N-Diethyl-, N,N-Dibutyl- und N,N-Diphenylamino)butyl, 6-(N,N-Dimethyl-, N,N-Diethyl-, N,N-Dibutyl- und N,N-Diphenylamino)hexyl, 8-(N,N-Dimethyl-, N,N-Diethyl-, N,N-Dibutyl- und N,N-Diphenylamino)octyl und 12-(N,N-Dimethyl-, N,N-Diethyl-, N,N-Dibutyl- und N,N-Diphenylamino)-dodecyl;
Sulfamidomethyl, 2-Sulfamidoethyl, 3-Sulfamidopropyl, 4-Sulfamidobutyl, 5-Sulfamidopentyl, 6-Sulfamidohexyl, 8-Sulfamidooctyl, 10-Sulfamidodecyl, 12-Sulfamidododecyl und 18-Sulfamidooctadecyl;
N,N-Dimethyl-, N,N-Diethyl-, N,N-Dibutyl- und N,N-Diphenylsulfamidomethyl, 2-(N,N-Dimethyl-, N,N-Diethyl-, N,N-Dibutyl- und N,N-Diphenylsulfamido)ethyl, 3-(N,N-Dimethyl-, N,N-Diethyl-, N,N-Dibutyl- und N,N-Diphenylsulfamido)propyl, 4-(N,N-Dimethyl-, N,N-Diethyl-, N,N-Dibutyl- und N,N-Diphenylsulfamido)butyl, 5-(N,N-Dimethyl-, N,N-Diethyl-, N,N-Dibutyl- und N,N-Diphenylsulfamido)pentyl, 6-(N,N-Dimethyl-, N,N-Diethyl-, N,N-Dibutyl- und N,N-Diphenylsulfamido)hexyl, 8-(N,N-Dimethyl-, N,N-Diethyl-, N,N-Dibutyl- und N,N-Diphenylsulfamido)octyl, 10-(N,N-Dimethyl-, N,N-Diethyl-, N,N-Dibutyl- und N,N-Diphenylsulfamido)decyl, 12-(N,N-Dimethyl-, N,N-Diethyl-, N,N-Dibutyl- und N,N-Diphenylsulfamido)dodecyl und 18-(N,N-Dimethyl-, N,N-Diethyl-, N,N-Dibutylund N,N-Diphenylsulfamido)octadecyl;
N,N-Dimethyl-, N,N-Diethyl-, N,N-Dibutyl- und N,N-Diphenylcarboxamidomethyl, 2-(N,N-Dimethyl-, N,N-Diethyl-, N,N-Dibutyl- und N,N-Diphenylcarboxamido)ethyl, 3-(N,N-Dimethyl-, N,N-Diethyl-, N,N-Dibutyl- und N,N-Diphenylcarboxamido)propyl, 4-(N,N-Dimethyl-, N,N-Diethyl-, N,N-Dibutyl- und N,N-Diphenylcarboxamido)butyl, 5-(N,N-Dimethyl-, N,N-Diethyl-, N,N-Dibutyl- und N,N-Diphenylcarboxamido)pentyl, 6-(N,N-Dimethyl-, N,N-Diethyl-, N,N-Dibutyl- und N,N-Diphenylcarboxamido)hexyl, 8-(N,N-Dimethyl-, N,N-Diethyl-, N,N-Dibutyl- und N,N-Diphenylcarboxamido)octyl, 10-(N,N-Dimethyl-, N,N-Diethyl-, N,N-Dibutyl- und N,N-Diphenylcarboxamido)decyl, 12-(N,N-Dimethyl-, N,N-Diethyl-, N,N-Dibutylund N,N-Diphenylcarboxamido)dodecyl und 18-(N,N-Dimethyl-, N,N-Diethyl-, N,N-Dibutyl- und N,N-Diphenylcarboxamido)octadecyl;
Methyl-, Ethyl-, Propyl-, Butyl-, Pentyl- und Hexylcarboxymethyl, Methyl-, Ethyl-, Propyl-, Butyl-, Pentyl- und Hexyl-2-carboxyethyl, Methyl-, Ethyl-, Propyl-, Butyl-, Pentyl- und Hexyl-3-carboxypropyl, Methyl-4-carboxybutyl, Methyl-5-carboxypentyl, Methyl-6-carboxyhexyl, Methyl-8-carboxyoctyl, Methyl-10-carboxydecyl, Methyl-12-carboxydodecyl und Methyl-14-carboxytetradecyl;
Cyclopentyl, 2- und 3-Methylcyclopentyl, 2- und 3-Ethylcyclopentyl, Cyclohexyl, 2-, 3- und 4-Methylcyclohexyl, 2-, 3- und 4-Ethylcyclohexyl, 3- und 4-Propylcyclohexyl, 3- und 4-Isopropylcyclohexyl, 3- und 4-Butylcyclohexyl, 3- und 4-sec.-Butylcyclohexyl, 3- und 4-tert.-Butylcyclohexyl, Cycloheptyl, 2-, 3- und 4-Methylcycloheptyl, 2-, 3- und 4-Ethylcycloheptyl, 3- und 4-Propylcycloheptyl, 3- und 4-Isopropylcycloheptyl, 3- und 4-Butylcycloheptyl, 3- und 4-sec.-Butylcycloheptyl, 3- und 4-tert.-Butylcycloheptyl, Cyclooctyl, 2-, 3-, 4- und 5-Methylcyclooctyl, 2-, 3-, 4- und 5-Ethylcyclooctyl, 3-, 4- und 5-Propylcyclooctyl, 1,3-Dioxan-2-yl, 1,4-Dioxan-2-yl, N-Methyl-, N-Ethyl-, N-Propyl-, N-Butyl-, N-Phenyl- und N-Benzyl-2-morpholinyl, N-Methyl-, N-Ethyl-, N-Propyl-, N-Butyl-, N-Phenyl- und N-Benzyl-3-morpholinyl, 2- und 3-Tetrahydrofuryl, 2- und 3-Tetrahydrothienyl, 1-, 2- und 3-Pyrrolidinyl und 1-, 2-, 3- und 4-Piperidyl;
Phenyl, 1- und 2-Naphthyl, 2- und 3-Pyrryl, 2-, 3- und 4-Pyridyl, 2-, 4- und 5-Pyrimidyl, 3-, 4- und 5-Pyrazolyl, 2-, 4- und 5-Imidazolyl, 2-, 4- und 5-Thiazolyl, 3-(1,2,4-Triazyl), 2-(1,3,5-Triazyl), 6-Chinaldyl, 3-, 5-, 6- und 8-Chinolinyl, 2-Benzoxazolyl, 2-Benzothiazolyl, 5-Benzothiadiazolyl, 2- und 5-Benzimidazolyl und 1- und 5-Isochinolyl;
2-, 3- und 4-Methylphenyl, 2,3-, 2,4-, 2,5-, 3,5- und 2,6-Dimethylphenyl, 2,4,6-, 2,3,4- und 2,3,5-Trimethylphenyl, 2-, 3- und 4-Ethylphenyl, 2,3-, 2,4-, 2,5-, 3,5- und 2,6-Diethylphenyl, 2,4,6-, 2,3,4- und 2,3,5-Triethylphenyl, 2-, 3- und 4-Propylphenyl, 2,3-, 2,4-, 2,5-, 3,5- und 2,6-Dipropylphenyl, 2,4,6-, 2,3,4- und 2,3,5-Tripropylphenyl, 2-, 3- und 4-Isopropylphenyl, 2,3-, 2,4-, 2,5-, 3,5- und 2,6-Diisopropylphenyl, 2,4,6-, 2,3,4- und 2,3,5-Triisopropylphenyl, 2-, 3- und 4-Butylphenyl, 2,3-, 2,4-, 2,5-, 3,5- und 2,6-Dibutylphenyl, 2,4,6-, 2,3,4- und 2,3,5-Tributylphenyl, 2-, 3- und 4-Isobutylphenyl, 2,3-, 2,4-, 2,5-, 3,5- und 2,6-Diisobutylphenyl, 2,4,6-, 2,3,4- und 2,3,5-Triisobutylphenyl, 2-, 3- und 4-tert.-Butylphenyl, 2,4-, 2,5-, 3,5- und 2,6-Di-tert.-butylphenyl; 2-, 3- und 4-Methoxyphenyl, 2,3-, 2,4-, 2,5-, 3,5- und 2,6-Dimethoxyphenyl, 2,4,6-, 2,3,4- und 2,3,5-Trimethoxyphenyl, 2-, 3- und 4-Ethoxyphenyl, 2,3-, 2,4-, 2,5-, 3,5- und 2,6-Diethoxyphenyl, 2,4,6-, 2,3,4- und 2,3,5-Triethoxyphenyl, 2-, 3- und 4-Propoxyphenyl, 2,3-, 2,4-, 2,5-, 3,5- und 2,6-Dipropoxyphenyl, 2,4,6-, 2,3,4- und 2,3,5-Tripropoxyphenyl, 2-, 3- und 4-Isopropoxyphenyl, 2,3-, 2,4-, 2,5-, 3,5- und 2,6-Diisopropoxyphenyl und 2,4,6-, 2,3,4- und 2,3,5-Triisopropoxyphenyl;
4-Phenylazophenyl, 4-(1-Naphthylazo)phenyl, 4-(2-Naphthylazo)phenyl, 4-(2-Pyridylazo)phenyl, 4-(3-Pyridylazo)phenyl, 4-(4-Pyridylazo)phenyl, 4-(2-Pyrimidylazo)phenyl, 4-(4-Pyrimidylazo)phenyl und 4-(5-Pyrimidylazo)phenyl;
Benzyl, Phenylethyl, Phenylpropyl, Phenylbutyl, Phenylpentyl, Phenylhexyl, 2-, 3- und 4-Methylbenzyl, 2-, 3- und 4-Ethylbenzyl, 2-, 3- und 4-Butylbenzyl, 2,3-, 2,4-, 2,5-, 3,5- und 2,6-Dimethylbenzyl, 2,3-, 2,4-, 2,5-, 3,5- und 2,6-Diethylbenzyl, 2-, 3- und 4-Methoxybenzyl, 2-, 3- und 4-Ethoxybenzyl, 2-, 3- und 4-Butoxybenzyl, 2,3-, 2,4-, 2,5-, 3,5- und 2,6-Dimethoxybenzyl, 2,3-, 2,4-, 2,5-, 3,5- und 2,6-Diethoxybenzyl, 4-Phenylazobenzyl, β-(2-, 3- und 4-Methylphenyl)ethyl, β-(2-, 3- und 4-Ethylphenyl)ethyl, β-(2-, 3- und 4-Butylphenyl)ethyl, β-(2,3-, 2,4-, 2,5-, 3,5- und 2,6-Dimethylphenyl)ethyl, β-(2,3-, 2,4-, 2,5-, 3,5-und 2,6-Diethylphenyl)ethyl, β-(2-, 3- und 4-Methoxyphenyl)ethyl, β-(2-, 3- und 4-Ethoxyphenyl)ethyl, β-(2-, 3- und 4-Butoxyphenyl)ethyl, β-(2,3-, 2,4-, 2,5-, 3,5- und 2,6-Dimethoxyphenyl)ethyl, β-(2,3-, 2,4-, 2,5-, 3,5- und 2,6-Diethoxyphenyl)ethyl und β-(4-Phenylazophenyl)ethyl; -
3- und 4-(N,N-Dimethylamino)phenyl, 3- und 4-(N,N-Diethyl-amino)phenyl, 3- und 4-(N,N-Dibutylamino)phenyl, 4-(N,N-Diphenyl-amino)phenyl, 3,5-Bis(N,N-Dimethylamino)phenyl, 3,5-Bis(N,N-Di-ethylamino)phenyl, 3,5-Bis(N,N-Dibutylamino)phenyl, 3,5-Bis(N,N-Diphenylamino) phenyl, 3- und 4-(N,N-Dimethylamino)benzyl, 3- und 4-(N,N-Diethylamino)benzyl, 3- und 4-(N,N-Dibutylamino)benzyl, 3- und 4-(N,N-Diphenylamino)benzyl, 3,5-Bis(N,N-Dimethyl-amino)benzyl, 3,5-Bis(N,N-Diethylamino)benzyl, 3,5-Bis(N,N-Dibu-tylamino)benzyl, 3,5-Bis(N,N-Diphenylamino)benzyl, β-[3- und 4-(N,N-Dimethylamino)phenyl]ethyl, β-[3- und 4-(N,N-Diethyl-amino)phenyl]ethyl, β-[3- und 4-(N,N-Dibutylamino)phenyl]ethyl, β-[4-(N,N-Diphenylamino)phenyl]ethyl, β-[3,5-Bis(N,N-Dimethyl-amino)phenyl]ethyl, β-[3,5-Bis(N,N-Diethylamino)phenyl]ethyl, β-[3,5-Bis(N,N-Dibutylamino)phenyl]ethyl, β-[3,5-Bis(N,N-Diphenyl-amino)phenyl]ethyl, 4-(N,N-Dimethylamino)naphth-1-yl, 4-(N,N-Di-ethylamino)naphth-1-yl, 4-(N,N-Dibutylamino)naphth-1-yl, 4-(N,N-Diphenylamino)naphth-1-yl, 5-(N,N-Dimethylamino)naphth-1-yl, 5-(N,N-Diethylamino)naphth-1-yl, 5-(N,N-Dibutylamino)naphth-1-yl, 5-(N,N-Diphenylamino)naphth-1-yl, 6-(N,N-Dimethylamino)naphth--2-yl, 6-(N,N-Diethylamino)naphth-2-yl, 6-(N,N-Dibutylamino)naphth-2-yl und 6-(N,N-Diphenylamino)naphth-2-yl;
3- und 4-(N,N-Dimethylcarboxamido)phenyl, 3- und 4-(N,N-Diethyl-carboxamido)phenyl, 3- und 4-(N,N-Dibutylcarboxamido)phenyl, 4-(N,N-Diphenylcarboxamido)phenyl, 3,5-Bis(N,N-dimethylcarboxamido)phenyl, 3,5-Bis(N,N-diethylcarboxamido)phenyl, 3,5-Bis(N,N-dibutylcarboxamido)phenyl, 3,5-Bis(N,N-diphenylcarboxamido)phenyl, 3- und 4-(N,N-Dimethylcarboxamido)benzyl, 3- und 4-(N,N-Diethylcarboxamido)benzyl, 3- und 4-(N,N-Dibutylcarboxamido)benzyl, 4-(N,N-Diphenylcarboxamido)benzyl, 3,5-Bis(N,N-dimethylcarboxamido)benzyl, 3,5-Bis(N,N-diethylcarboxamido)benzyl, 3,5-Bis(N,N-dibutylcarboxamido)benzyl, 3,5-Bis(N,N-diphenylcarboxamido)benzyl, β-[3- und 4-(N,N-Dimethylcarboxamido)phenyl]-ethyl, β-[3- und 4-(N,N-Diethylcarboxamido)phenyl]ethyl, β-[3- und 4-(N,N-Dibutylcarboxamido)phenyl]ethyl, β-[4-(N,N-Diphenylcarboxamido)phenyl]ethyl, β-[3,5-Bis(N,N-dimethylcarboxamido)phenyl]ethyl, β-[3,5-Bis(N,N-diethylcarboxamido)phenyl]ethyl, β-[3,5-Bis(N,N-dibutylcarboxamido)phenyl]ethyl und β-[3,5-Bis(N,N-diphenylcarboxamido)phenyl]ethyl;
2-, 3- und 4-(Carboxymethyl)phenyl, 2-, 3- und 4-(Carboxyethyl)-phenyl, 2-, 3- und 4-(Carboxybutyl)phenyl, 3- und 4-(Carboxyphenyl)phenyl, 2,4-, 2,5- und 3,5-Bis(carboxymethyl)phenyl, 2,4-, 2,5- und 3,5-Bis(carboxyethyl)phenyl, 2,4-, 2,5- und 3,5-Bis(carboxybutyl)phenyl, 3,5-Bis(carboxyphenyl)phenyl, 2-, 3- und 4-(Carboxymethyl)benzyl, 2-, 3- und 4-(Carboxyethyl)benzyl, 2-, 3- und 4-(Carboxybutyl)benzyl, 3- und 4-(Carboxyphenyl)benzyl, 2,4-, 2,5- und 3,5-Bis(carboxymethyl)benzyl, 2,4-, 2,5- und 3,5-Bis(carboxyethyl)benzyl, 2,4-, 2,5- und 3,5-Bis (carboxybutyl)benzyl, 3,5-Bis(carboxyphenyl)benzyl, β-[2-, 3- und 4-(Carboxymethyl)phenyl]ethyl, β-[2-, 3- und 4-(Carboxyethyl)phenyl]-ethyl, β-[2-, 3- und 4-(Carboxybutyl)phenyl]ethyl, β-[3- und 4-(Carboxyphenyl)phenyl]ethyl, β-[2,4- 2,5- und 3,5-Bis(carboxymethyl)phenyl]ethyl, β-[2,4-, 2,5- und 3,5-Bis(carboxyethyl)phenyl]ethyl, β-[2,4-, 2,5- und 3,5-Bis(carboxybutyl)phenyl]ethyl, β-[3,5-Bis(carboxyphenyl)phenyl]ethyl, 2-, 4- und 5-(Carboxymethyl)naphth-1-yl, 2-, 4- und 5-(Carboxyethyl)naphth-1-yl, 2-, 4-und 5-(Carboxybutyl)naphth-1-yl, 2-, 4- und 5-(Carboxyphenyl) naphth-1-yl, 4-, 5-, 6-, 7- und 8-(Carboxymethyl)naphth-2-yl, 4-, 5-, 6-, 7- und 8-(Carboxyethyl)naphth-2-yl, 4-, 5-, 6-, 7- und 8-(Carboxybutyl)naphth-2-yl und 4-, 5-, 6-, 7- und 8-(Carboxyphenyl)naphth-2-yl.

Beim erfindungsgemäßen Verfahren zur Herstellung der Perylimide I werden die entsprechend substituierten Naphthalimide II in Gegenwart eines unpolar-aprotischen organischen Lösungsmittels und einer alkalimetallhaltigen Base dimerisiert (Schritt a), und die dabei als Alkalimetallsalz anfallende Leukoform des Perylimids I ("Küpensalz") wird anschließend in Gegenwart eines polaren Lösungsmittels reoxidiert (Schritt b).

Als Lösungsmittel sind in Schritt a) grundsätzlich alle unter den Reaktionsbedingungen gegen Basen stabilen unpolar-aprotischen Lösungsmittel mit einem Siedepunkt oberhalb der gewählten Reaktions-temperatur geeignet, in denen sich die Naphthalimide II bei Reaktionstemperatur vollständig und die verwendeten Basen zumindest partiell lösen, so daß weitgehend homogene Reaktionsbedingungen vorliegen.

Beispiele für bevorzugte Lösungsmittel sind bei > 100°C siedende Lösungsmittel aus den folgenden Gruppen: Aliphaten (insbesondere C₈-C₁₈-Alkane), unsubstituierte, alkylsubstituierte und kondensierte Cycloaliphaten (insbesondere unsubstituierte C₇-C₁₀-Cycloalkane, C₆-C₈-Cycloalkane, die durch ein bis drei C₁-C₆-Alkylgruppen substituiert sind, polycyclische gesättigte Kohlenwasserstoffe mit 10 bis 18 C-Atomen), alkyl-und cycloalkylsubstituierte Aromaten (insbesondere Benzol, das durch ein bis drei C₁-C₆-Alkylgruppen oder einen C₅-C₈-Cycloalkylrest substituiert ist) und kondensierte Aromaten, die alkylsubstituiert und/oder teilhydriert sein können (insbesondere Naphthalin, das durch ein bis vier C₁-C₆-Alkylgruppen substituiert ist) sowie Mischungen dieser Lösungsmittel.

Als Beispiele für besonders bevorzugte Lösungsmittel seien im einzelnen genannt: Octan, Isooctan, Nonan, Isononan, Decan, Isodecan, Undecan, Dodecan, Hexadecan und Octadecan; Cycloheptan, Cyclooctan, Methylcyclohexan, Dimethylcyclohexan, Trimethylcyclohexan, Ethylcyclohexan, Diethylcyclohexan, Propylcyclohexan, Isopropylcyclohexan, Dipropylcyclohexan, Butylcyclohexan, tert.-Butylcyclohexan, Methylcycloheptan und Methylcyclooctan; Toluol, o-, m- und p-Xylol, 1,3,5-Trimethylbenzol (Mesitylen), 1,2,4- und 1,2,3-Trimethylbenzol, Ethylbenzol, Propylbenzol, Isopropylbenzol, Butylbenzol, Isobutylbenzol, tert.-Butylbenzol und Cyclohexylbenzol; Naphthalin, Decahydronaphthalin (Dekalin), 1- und 2-Methylnaphthalin, 1- und 2-Ethylnaphthalin; Kombinationen aus den zuvor genannten Lösungsmitteln, wie sie aus den hochsiedenden, teil- oder durchhydrierten Fraktionen thermischer und katalytischer Crackprozesse bei der Rohöl- oder Naphthaverarbeitung gewonnen werden können, z.B. Gemische vom Exxsol^{®} Typ, und Alkylbenzolgemische vom Solvesso^{®} Typ.

Ganz besonders bevorzugte Lösungsmittel sind Isopropylcyclohexan, Dimethylcyclohexan (alle Isomeren), Trimethylcyclohexan (alle Isomeren), Dekalin, Xylol (alle Isomeren) und Mesitylen.

Als Base sind in Schritt a) starke anorganische und organische alkalimetallhaltige Basen mit geringer nucleophiler Wirkung geeignet. Bevorzugte anorganische Basen sind Alkalimetallhydroxide und -amide, bevorzugte organische Basen sind Alkalimetallalkoholate (insbesondere die C₁-C₅-Alkoholate), Alkalimetall(phenyl)alkylamide (insbesondere die Bis(C₁-C₄-alkyl)amide) und Triphenylmethylmetallate. Bevorzugte Alkalimetalle sind Lithium, Natrium und Kalium, wobei Kalium besonders bevorzugt ist.

Als Beispiele für besonders bevorzugte Basen seien im einzelnen genannt: Lithiumhydroxid, Natriumhydroxid und Kaliumhydroxid; Lithiumamid, Natriumamid und Kaliumamid; Lithiummethylat, Natriummethylat, Kaliummethylat, Lithiumethylat, Natriumethylat, Kaliumethylat, Natriumisopropylat, Kaliumisopropylat, Natrium-tert.-butylat und Kalium-tert.-butylat; Lithiumdimethylamid, Lithiumdiethylamid, Lithiumdiisopropylamid, Natriumdiisopropylamid, Triphenylmethyllithium, Triphenylmethylnatrium und Triphenylmethylkalium.

Ganz besonders bevorzugte Basen sind Lithiumdiisopropylamid, Natriummethylat, Natrium-tert.-butylat und vor allem Kalium-tert.-butylat, Kaliummethylat und Kaliumhydroxid.

Bei Verwendung der Methylate und der Hydroxide empfiehlt sich zur Erhöhung der Reaktivität der Zusatz geringer Mengen eines stickstoffhaltigen, 5 bis 6 Ringatome aufweisenden, als Phasenvermittler wirkenden Heterocyclus wie Pyridin, N-Methylpiperidin, N-Methylpiperidon, N-Methylmorpholin oder insbesondere N-Methyl-2-pyrrolidon. Geeignete Einsatzmengen liegen hier im allgemeinen bei 5 bis 20 Gew.-%, bezogen auf das Naphthalimid II.

Von der Alkalimetallbase werden in der Regel 1,8 bis 8 molare Äquivalente, vorzugsweise 1,8 bis 2,5 molare Äquivalente bei den organischen Basen und 2 bis 5 molare Äquivalente bei den anorganischen Basen, bezogen auf das Naphthalimid II, eingesetzt.

Die Alkalimetallbase kann in fester oder in gelöster Form eingesetzt werden. Wenn die Alkalimetallbase in dem unpolar-aprotischen Reaktionslösungsmittel nicht ausreichend löslich ist, kann sie in einem Alkohol, der eine höhere Basenstärke als die Alkalimetallbase hat, gelöst werden. Geeignet sind vor allem tertiäre aliphatische Alkohole, die Arylsubstituenten enthalten können und insgesamt vier bis zwölf C-Atome aufweisen, z.B. tert.-Butanol, 2-Methyl-2-butanol (tert.-Amylalkohol), 3-Methyl-3-pentanol, 3-Ethyl-3-pentanol, 2-Phenyl-2-pentanol, 2,3-Dimethyl-3-pentanol, 2,4,4-Trimethyl-2-pentanol und 2,2,3,4,4-Pentamethyl-3-pentanol.

Die Lösungsmittelmenge hängt beim Verfahrensschritt a) von der Art der Reaktionsführung ab. Wie weiter unten ausgeführt, ist sowohl eine diskontinuierliche als auch eine quasikontinuierliche Fahrweise möglich.

Bei diskontinuierlicher (Batch-) Fahrweise muß zumindest das Naphthalimid II bei Reaktionstemperatur vollständig gelöst sein. Deshalb werden üblicherweise 5 bis 50 kg, vorzugsweise 7 bis 25 kg, Lösungsmittel je kg II eingesetzt. Wenn die Alkalimetallbase als Lösung zugegeben wird, werden zusätzlich in der Regel 3 bis 10 kg Lösungsmittel je kg Base benötigt. Wenn die Alkalimetallbase als Feststoff verwendet wird, besteht im allgemeinen kein weiterer Lösungsmittelbedarf.

Bei quasikontinuierlicher Fahrweise müssen sowohl das Naphthalimid II als auch die Alkalimetallbase bei der Reaktionstemperatur vollständig gelöst sein. Die erforderliche Gesamtlösungsmittelmenge erhöht sich daher auf in der Regel 8 bis 100 kg, bevorzugt 10 bis 50 kg. Dabei bleibt die für das Naphthalimid II benötigte Menge gleich, und für die Base werden je kg im allgemeinen 3 bis 50 kg, vorzugsweise 3 bis 25 kg, Lösungsmittel benötigt.

Die Reaktionstemperatur beträgt beim Verfahrensschritt a) üblicherweise 80 bis 250°C, wobei die bevorzugten Reaktionstemperaturen durch die Reaktivität und die Löslichkeit der eingesetzten Naphthalimide II bestimmt werden. So sind bei den hochreaktiven N-alkylsubstituierten Naphthalimiden II Temperaturen von 80 bis 150°C, insbesondere 100 bis 130°C, bevorzugt. Bei gut löslichen, basenstabilen N-arylsubstituierten Naphthalimiden II mittlerer Reaktivität liegt die Reaktionstemperatur bevorzugt bei 130 bis 200°C, vor allem bei 150 bis 180°C, während für schlecht lösliche, basenlabile oder reaktionsträge Naphthalimide II eine Reaktionstemperatur von 170 bis 250°C, vorzugsweise 180 bis 210°C, besonders geeignet ist. Durch die gegenüber den bekannten Herstellungsverfahren drastisch verkürzten Reaktionszeiten ist auch bei den basenlabilen Naphthalimiden II trotz der hohen Reaktionstemperaturen eine deutlich verringerte Zersetzung zu beobachten.

So beträgt die Reaktionszeit bei diskontinuierlicher Fahrweise im allgemeinen 0,1 bis 10 h, vorzugsweise 0,2 bis 6 h bzw. 0,1 bis 1 h bei basenlabilen Naphthalimiden II, und bei quasikontinuierlicher Fahrweise in der Regel 5 bis 1200 sec, vorzugsweise 5 bis 300 sec.

Üblicherweise wird die Dimerisierung bei diskontinuierlicher Fahrweise unter Normaldruck durchgeführt. Soll die Reaktionstemperatur über dem Siedepunkt des Lösungsmittels liegen, kann man selbstverständlich auch im geschlossenen System unter dem sich entwickelnden Eigendruck oder unter Druckregelung arbeiten. Bei quasikontinuierlicher Fahrweise wird üblicherweise unter einem Druck von etwa 1 bis 50 bar dimerisiert.

Verfahrenstechnisch geht man in Schritt a) des erfindungsgemäßen Verfahrens zur Herstellung der Perylimide I zweckmäßigerweise wie folgt vor:

Bei diskontinuierlicher Fahrweise löst man Naphthalimid II und gegebenenfalls den stickstoffhaltigen Reaktionsvermittler unter Erwärmen im Lösungsmittel, erhitzt unter Stickstoff und Rühren auf die gewünschte Reaktionstemperatur, gibt dann in etwa 5 bis 60 min die Alkalimetallbase portionsweise (als Feststoff) oder in 0,2 bis 6 h kontinuierlich (gelöst) zu und rührt weitere 2 bis 60 min bei der Reaktionstemperatur nach. Nach Abkühlen auf 20 bis 80°C filtriert man das ausgefallene Produkt unter Stickstoff ab und setzt es dann nach kurzer Wäsche entweder sofort feucht in Schritt b) zur Reoxidation ein, oder man extrahiert das Küpensalz mit einem geeigneten polaren Lösungsmittel, wie Wasser, Methanol, Ethanol oder Eisessig, unter Inertbedingungen aus dem Filterkuchen und setzt die erhaltene Extraktionslösung in Schritt b) ein. Die letztgenannte Vorgehensweise wird vorzugsweise bei der Umsetzung von arylsubstituierten Naphthalimiden II eingesetzt, da die entsprechenden Küpensalze grundsätzlich als im Reaktionsmedium unlösliche, kristalline Addukte mit unumgesetztem Naphthalimid im Verhältnis 1:2 anfallen und auf diese Weise eine aufwendige Endreinigung der gebildeten Perylimide I vermieden werden kann.

Bei quasikontinuierlicher Fahrweise bringt man sauerstofffreie, auf Reaktionstemperatur erhitzte Lösungen von Naphthalimid II und Alkalimetallbase in demselben Lösungsmittel in einem Mischkammer-oder Rohrreaktor bei einem Druck von etwa 1 bis 50 bar zur Reaktion, wobei man die Dosierrate der beiden Lösungen so wählt, daß sich Reaktorverweilzeiten von etwa 5 bis 1200 sec ergeben. Die aus dem Reaktor austretende Reaktionsmischung kühlt man dann schnell auf 20 bis 80°C ab und arbeitet das ausgefallene Reaktionsprodukt, wie bei der diskontinuierlichen Fahrweise beschrieben, auf.

Die Reoxidation des als Küpensalz vorliegenden Perylimids I (Schritt b) des erfindungsgemäßen Verfahrens) wird in Gegenwart eines polaren Lösungsmittels vorgenommen.

Hierfür eignen sich alle polaren Lösungsmittel, in denen das Küpensalz löslich ist und das gebildete Perylimid I stabil ist.

Geeignet sind z.B. polar-protische anorganische Lösungsmittel wie Wasser und verdünnte wäßrige Lösungen anorganischer Säuren sowie polar-protische organische Lösungsmittel wie Alkohole, insbesondere C₁-C₆-Alkanole, und organische Säuren, insbesondere aliphatische Mono- und Dicarbonsäuren mit ein bis sechs C-Atomen.

Die Reoxidation kann auch in polar-aprotischen organischen Lösungsmitteln, z.B. heterocyclischen Stickstoffbasen, durchgeführt werden. In diesem Fall können die gebildeten Perylimide I durch Zugabe von protischen Lösungsmitteln wie Methanol zur Isolierung ausgefällt werden.

Selbstverständlich können auch Gemische der genannten Lösungsmittel eingesetzt werden.

Als bevorzugte Lösungsmittel seien im einzelnen genannt: Wasser; etwa 5 gew.-%ige Salzsäure; Methanol, Ethanol, Propanol, Isopropanol, Butanol und Hexanol; Ameisensäure, Essigsäure, Propionsäure, Buttersäure und Adipinsäure; N-Methyl-2-pyrrolidon.

Die Anwesenheit einer Säure empfiehlt sich insbesondere bei der Reoxidation zur Verseifung neigender, basenlabiler Perylimide I. Wird nicht die Säure selbst als Lösungsmittel verwendet, so wird dem Reaktionsgemisch üblicherweise so viel Säure zugegeben, daß ein pH-Wert von 2 bis 7 vorliegt.

Die Lösungsmittelmenge ist an sich unkritisch. Normalerweise werden 10 bis 80 kg Lösungsmittel je kg ursprünglich eingesetztes Naphthalimid II verwendet.

Als Oxidationsmittel werden vorzugsweise Sauerstoff (Luft) oder wäßrige, insbesondere etwa 5 bis 30 gew.-%ige, Wasserstoffperoxidlösungen im leichten Überschuß eingesetzt.

Die Reaktionstemperatur beträgt üblicherweise 20 bis 100°C, bevorzugt 30 bis 80°C.

Die Reaktionszeit liegt in Abhängigkeit von der Reaktionstemperatur in der Regel bei 1 bis 16 h. Bei 60 bis 65°C werden normalerweise 1 bis 3 h zur Oxidation benötigt und bei 30 bis 40°C etwa 4 bis 10 h.

Verfahrenstechnisch geht man in Schritt b) des erfindungsgemäßen Verfahrens zur Herstellung der Perylimide I zweckmäßigerweise wie folgt vor:
Man trägt das in Schritt a) erhaltene Küpensalz entweder in Form des feuchten Filterpreßkuchens unter Rühren in das Lösungsmittel ein oder löst es, wie oben beschrieben, mit Hilfe des Lösungs-mittels direkt vom Filter herunter, stellt erforderlichenfalls, wie oben beschrieben, einen neutralen bis sauren pH-Wert ein, erhitzt auf die gewünschte Reaktionstemperatur und leitet bei dieser Temperatur Luft ein oder gibt Wasserstoffperoxidlösung zur vollständigen Oxidation zu (erkennbar an einem Farbumschlag von schwarzviolett nach orangerot bis rot, je nach dargestelltem Perylimid I). Anschließend filtriert man das ausgefallene Endprodukt ab, wäscht mit dem verwendeten Lösungsmittel oder Wasser gründlich aus und trocknet. Gegebenenfalls kann man das gewaschene Produkt einer zusätzlichen Behandlung zur Entfernung nichtumgesetzten Naphthalimids unterziehen, indem man es 2 bis 8 h unter Rühren in der 5- bis 10-fachen Menge Eisessig unter Rückfluß erhitzt, heiß filtriert, mit Methanol oder Wasser neutral wäscht und dann trocknet.

Mit Hilfe des erfindungsgemäßen Verfahrens können die Perylimide I auf einfache, wirtschaftliche Weise in hohen Ausbeuten (üblicherweise > 75%, bezogen auf den Umsatz; bei alkyl- und cycloalkylsubstituierten Naphthalimiden II ist der Umsatz nahezu quantitativ, bei arylsubstituierten Naphthalimiden II liegt der Umsatz aufgrund der oben angesprochenen Adduktbildung des intermediär gebildeten Küpensalzes mit unumgesetztem Naphthalimid grundsätzlich bei maximal 50%) hergestellt werden. Auch die bislang nicht über Dimerisierung der entsprechenden Naphthalimide zugänglichen Perylimide (z.B. solche, die an den Imidstickstoffatomen sterisch stark gehinderte Substituenten wie 2,6-Diisopropylphenyl, 2,5- oder 2,6-Di-tert.-butylphenyl tragen) sind problemlos zu erhalten.

Die chemische Reinheit der erfindungsgemäß hergestellten Perylimide I liegt in der Regel bereits bei > 90%. Falls für bestimmte Anwendungen gewünscht, kann ihre Reinheit durch weitere, für derartige Verbindungen übliche Reinigungsschritte wie Rekristallisation aus Halogenkohlenwasserstoffen sowie Halogen- oder Nitroaromaten, Extraktion mit polar-aprotischen organischen Lösungsmitteln wie Isobutylmethylketon oder Behandlung mit einem Reduktionsmittel wie Natriumdithionit unter wäßrig-basischen Bedingungen mit anschließender Reoxidation der so gebildeten Küpensalze mit Sauerstoff unter moderaten Ausbeuteverlusten (etwa 10 bis 20%) auf > 98% erhöht werden. Die so gewonnenen hochreinen Perylimide I zeichnen sich durch besondere physikalische Eigenschaften, z.B. ausgeprägte Festkörperfluoreszenz, aus und sind damit für spezielle Anwendungen, z.B. als Elektrolumineszenzmaterialien und als Ladungserzeugungs- und/oder Ladungstransportverbindungen, geeignet. Die nach den herkömmlichen Verfahren hergestellten Perylimide weisen diese Eigenschaften nicht auf, da sie verfahrensbedingt immer durch farbige, fluoreszenzlöschende, auch über aufwendige Reinigungsverfahren nicht abtrennbare Perylenderivate verunreinigt sind.

Wie bereits erwähnt, kann durch Modifizierung der Reoxidation des Küpensalzes (Schritt b) des erfindungsgemäßen Verfahrens zur Herstellung der Perylimide I) vorteilhaft auch Perylen-3,4:9,10-tetracarbonsäuredianhydrid erhalten werden.

Bei diesem ebenfalls erfindungsgemäßen Herstellungsverfahren werden im analog vorgenommenen Schritt a) vorzugsweise solche Naphthalimide (IIa) eingesetzt, die am Imidstickstoff die Reste R³ (Cyclohexyl oder Phenyl, das jeweils auch durch bis zu drei C₁-C₄-Alkylreste substituiert sein kann, wie 2-, 3- und 4-Methylcyclohexyl, 2-, 3- und 4-Ethylcyclohexyl, 3- und 4-Propylcyclohexyl, 3- und 4-Isopropylcyclohexyl, 3- und 4-Butylcyclohexyl, 3- und 4-sec.-Butylcyclohexyl, 3- und 4-tert.-Butylcyclohexyl, Phenyl, 3- und 4-Methylphenyl, 2,3-, 2,4-, 2,5- und 3,5-Dimethylphenyl, 2,3,4- und 2,3,5-Trimethylphenyl, 3- und 4-Ethylphenyl, 3,5-Diethylphenyl, 3- und 4-Propylphenyl, 3,5-Dipropylphenyl, 3-und 4-Isopropylphenyl, 3,5-Diisopropylphenyl, 3- und 4-Butylphenyl und 3,5-Dibutylphenyl) tragen.

In Schritt b) des erfindungsgemäßen Verfahrens zur Herstellung von Perylen-3,4:9,10-tetracarbonsäuredianhydrid, der in Gegenwart eines inerten Lösungsmittels, einer Base und von Wasser durchgeführt wird, wird die Reoxidation mit einer Verseifung gekoppelt.

Als Lösungsmittel können dabei alle Lösungsmittel eingesetzt werden, die basenstabil sind und in denen das Küpensalz zumindest partiell löslich ist.

Geeignet sind beispielsweise polar-protische Lösungsmittel wie aliphatische Alkohole, insbesondere C₃-C₆-Alkanole, und basenstabile unpolar-aprotische Lösungsmittel, insbesondere Aromaten wie alkylsubstituierte Benzole und kondensierte Cycloalkane, wobei die Alkohole bevorzugt sind. Selbstverständlich können auch Mischungen dieser Lösungsmittel eingesetzt werden.

Im einzelnen seien als bevorzugte Lösungsmittel z.B. genannt: Propanol, Isopropanol, Butanol, sec.-Butanol, tert.-Butanol, Pentanol, 2-Methyl-2-butanol, 3-Methyl-3-pentanol und Hexanol; Toluol, o-, m- und p-Xylol und 1,3,5-, 1,2,4- und 1,2,3-Trimethylbenzol.

Die Lösungsmittelmenge ist an sich unkritisch. Normalerweise werden 10 bis 80 kg Lösungsmittel je kg ursprünglich eingesetztes Naphthalimid IIa verwendet.

Als Base sind anorganische und organische alkalimetallhaltige Basen geeignet. Bevorzugte anorganische Basen sind Alkalimetallhydroxide, bevorzugte organische Basen sind Alkalimetallalkoholate (insbesondere die C₁-C₄-Alkoholate). Bevorzugte Alkalimetalle sind Lithium, Natrium und Kalium, wobei Kalium besonders bevorzugt ist. Selbstverständlich können auch Gemische dieser Basen eingesetzt werden.

Als Beispiele für besonders bevorzugte Basen seien im einzelnen genannt: Lithiumhydroxid, Natriumhydroxid und Kaliumhydroxid; Lithiummethylat, Natriummethylat, Kaliummethylat, Lithiumethylat, Natriumethylat, Kaliumethylat, Natriumpropylat, Kaliumpropylat, Natriumisopropylat, Kaliumisopropylat, Natriumbutylat, Kaliumbutylat, Natrium-sec.-butylat, Kalium-sec.-butylat, Natrium-tert.-butylat und Kalium-tert.-butylat.

In der Regel kommen 2 bis 4 kg Alkalimetallhydroxid je kg ursprünglich eingesetztes Naphthalimid IIa bzw. 2 bis 8 molare Äquivalente Alkalimetallalkoholat, bezogen auf IIa, zum Einsatz.

Als Oxidationsmittel werden vorzugsweise Sauerstoff (Luft) oder wäßrige, insbesondere etwa 5 bis 30 gew.-%ige, Wasserstoffperoxidlösungen im Überschuß eingesetzt, wobei Luft in Kombination mit Alkalimetallalkoholaten und Wasserstoffperoxid in Kombination mit Alkalimetallhydroxiden bevorzugt ist.

Die Anwesenheit von Wasser in zumindest stöchiometrischen Mengen ist für die Vervollständigung der Verseifungsreaktion essentiell.

Bei Verwendung von Alkalimetallhydroxiden als Base in polarem Lösungsmittel werden, unabhängig vom verwendeten Oxidationsmittel, vorzugsweise 50 bis 100 mol Wasser und in unpolarem Lösungsmittel 2 bis 20 mol Wasser je mol ursprünglich eingesetztes Naphthalimid IIa zugesetzt.

Bei Verwendung von Alkalimetallalkoholaten als Base ist der Wasserbedarf unanbhängig vom eingesetzten Lösungmittel. Der Zusatz von Wasser erübrigt sich, wenn wäßrige Wasserstoffperoxidlösungen als Oxidationsmittel dienen, bei Oxidation mit Luft werden bevorzugt stöchiometrische Mengen Wasser (d.h., im allgemeinen 0,8 bis 1,2 mol Wasser je mol IIa), vorzugsweise kontinuierlich und gleichzeitig mit der Lufteinleitung, zugesetzt.

Die Reaktionstemperatur liegt üblicherweise bei 50 bis 180°C, bevorzugt bei 70 bis 140°C.

Die Reaktionszeit hängt vom verwendeten Lösungsmittel und der Reaktionstemperatur ab und beträgt in polaren Lösungsmitteln in der Regel 3 bis 10 h, vorzugsweise 4 bis 6 h, und in unpolaren Lösungsmitteln im allgemeinen 0,1 bis 2 h, vorzugsweise 0,1 bis 0,5 h.

Verfahrenstechnisch geht man in Schritt b) des erfindungsgemäßen Verfahrens zur Herstellung von Perylen-3,4:9,10-tetracarbonsäuredianhydrid zweckmäßigerweise wie folgt vor:
Man trägt das in Schritt a) erhaltene Küpensalz entweder in Form des feuchten Filterpreßkucheris unter Rühren in das Lösungsmittel ein oder löst es mit Hilfe des Lösungsmittels direkt vom Filter herunter, gibt Base und gegebenenfalls Wasser zu, erhitzt auf die gewünschte Reaktionstemperatur und leitet bei dieser Temperatur Luft ein (gegenenfalls unter gleichzeitiger kontinuierlicher Zudosierung von stöchiometrischen Wassermengen) oder gibt Wasserstoffperoxidlösung zu. Der Fortgang der Reaktion ist am Farbumschlag von schwarzviolett über tiefrot nach gelbbraun erkennbar. Nach Abkühlen auf Raumtemperatur filtriert man das Alkalimetallsalz der Perylen-3,4:9,10-tetracarbonsäure ab und wäscht mit einem Alkohol wie Isopropanol oder Propanol neutral und trocknet. Zur Überführung in das Dianhydrid trägt man das Salz in die 30- bis 100-fache Menge verdünnter wäßriger anorganischer Säure, z.B. 5 bis 10 gew.-%iger Salzsäure, ein, kocht kurz auf, filtriert nach dem Abkühlen ab, wäscht mit Wasser neutral und trocknet.

Mit Hilfe dieses erfindungsgemäßen Verfahrens kann Perylen-3,4:9,10-tetracarbonsäuredianhydrid vorteilhaft auf einfache, wirtschaftliche Weise in hohen Ausbeuten (üblicherweise > 70%, analog zur Herstellung der Perylimide I bezogen auf den Umsatz) und in hohen Reinheiten (im allgemeinen > 95%) hergestellt werden.

Die als Ausgangsprodukt für die erfindungsgemäßen Verfahren zur Herstellung der Perylimide I und von Perylen-3,4:9,10-tetracarbonsäuredianhydrid dienenden Naphthalimide II können vorteilhaft nach dem ebenfalls erfindungsgemäßen Verfahren, bei dem Naphthalin-1,8-dicarbonsäureanhydrid in Gegenwart eines polar-aprotischen organischen Lösungsmittels sowie einer organischen oder anorganischen Säure oder eines sauren Übergangsmetallsalzes als Katalysator oder in Gegenwart von Phenol mit primären Aminen umgesetzt wird.

Als Lösungsmittel eignen sich dabei polar-aprotische organische Lösungsmittel wie N,N-disubstituierte aliphatische Carbonsäureamide, insbesondere N,N-Di-C₁-C₄-alkyl-C₁-C₄-carbonsäureamide, und stickstoffhaltige Heterocyclen. Ebenfalls als Lösungsmittel geeignet ist Phenol. Selbstverständlich können auch Gemische dieser Lösungsmittel eingesetzt werden. Wird Phenol als einziges Lösungsmittel verwendet, so reicht seine Säurewirkung in der Regel aus, und es muß kein zusätzlicher saurer Katalysator zugesetzt werden.

Als Beispiele für bevorzugte Lösungsmittel seien genannt: N,N-Dimethylformamid, N,N-Diethylformamid, N,N-Dimethylacetamid und N,N-Dimethylbutyramid; N-Methyl-2-pyrrolidon, Chinolin, Isochinolin, Chinaldin, Pyrimidin, N-Methylpiperidin und Pyridin; Phenol.

Besonders bevorzugte Lösungsmittel sind N,N-Dimethylacetamid, N-Methyl-2-pyrrolidon und Phenol.

Die Lösungsmittelmenge ist an sich unkritisch. Normalerweise werden 2 bis 6 kg Lösungsmittel je kg Naphthalin-1,8-dicarbonsäureanhydrid verwendet.

Als saurer Katalysator eignen sich organische Säuren, insbesondere aliphatische C₁-C₆-Mono- und Dicarbonsäuren wie Essigsäure, Propionsäure und Adipinsäure, aromatische Carbon- und Sulfonsäuren wie Benzoesäure, Benzolsulfonsäure und o-, m- und p-Toluolsulfonsäure und anorganische Säuren wie Schwefelsäure und Phosphorsäure, die bevorzugt in möglichst konzentrierter, wasserfreier Form eingesetzt werden, sowie organische und anorganische Salze von Übergangsmetallen wie Zink, Eisen und Kupfer, z.B. Zinkacetat, Zinkpropionat, Zinkoxid, Eisen(II)acetat, Eisen(III)-chlorid, Eisen(II)sulfat, Kupfer(I)oxid, Kupfer(II)acetat und Kupfer(II)sulfat. Selbstverständlich kann man auch Mischungen der genannten Katalysatoren verwenden.

In der Regel werden 5 bis 80 Gew.-% saurer Katalysator, bezogen auf Naphthalin-1,8-dicarbonsäureanhydrid, eingesetzt. Bevorzugte Mengen betragen bei den Säuren 20 bis 60 Gew.-% und bei den Übergangsmetallsalzen 10 bis 40 Gew.-%, jeweils bezogen auf Naphthalin-1,8-dicarbonsäureanhydrid.

Das Molverhältnis primäres Amin III/Naphthalin-1,8-dicarbonsäureanhydrid liegt im allgemeinen bei 1:1 bis 3:1, vorzugsweise bei 1:1 bis 1,5:1.

Die Reaktionstemperatur beträgt üblicherweise 0 bis 250°C, insbesondere 0 bis 80°C bei reaktiven aliphatischen Aminen, 80 bis 160°C bei (cyclo)aliphatischen und aromatischen Aminen mittlerer Aktivität und 140 bis 250°C bei reaktionsträgen aromatischen und heteroaromatischen Aminen. Bei Temperaturen oberhalb von 120°C wird zweckmäßigerweise unter Schutzgas wie Stickstoff gearbeitet.

Man kann die Umsetzung von Naphthalin-1,8-dicarbonsäuredianhydrid und primärem Amin bei Normaldruck oder bei einem Überdruck von üblicherweise bis zu 10 bar durchführen. Die Arbeitsweise unter Druck ist vor allem beim Einsatz flüchtiger Amine (Siedepunkt ≤ Reaktionstemperatur) zweckmäßig.

Die Reaktionszeit liegt in der Regel bei 0,5 bis 15 h, vorzugsweise bei 1 bis 10 h.

Verfahrenstechnisch geht man beim erfindungsgemäßen Verfahren zur Herstellung der Naphthalimide II wie folgt vor:
Man erhitzt die Mischung aus Naphthalin-1,8-dicarbonsäureanhydrid, Amin, Lösungsmittel und Katalysator unter Stickstoff etwa 0,5 bis 15 h auf die gewünschte Reaktionstemperatur. Nach Abkühlen auf Raumtemperatur filtriert man das ausgefallene Reaktionsprodukt ab, wäscht mit kaltem Lösungsmittel oder einem aliphatischen Alkohol wie Methanol und trocknet.

Soll die Umsetzung unter Druck vorgenommen werden, so verwendet man eine Druckapparatur als Reaktionsgefäß, auf die man nach dem Einfüllen der Komponenten einen Stickstoffdruck von etwa 1 bis 2 bar gibt, erhitzt anschließend die gewünschte Zeit auf die Reaktiontemperatur und entspannt nach dem Abkühlen.

Durch Versetzen der Mutterlauge mit der 1- bis 3-fachen Menge Methanol kann man in beiden Fällen eine weitere Produktfraktion mit geringerer Reinheit gewinnen. Bei einigen Naphthalimiden II kann man die Ausbeute deutlich steigern, indem man die Reäktionsmischung nach beendeter Umsetzung bei 90 bis 100°C langsam mit etwa der 5-fachen Menge einer verdünnten anorganischen Säure, z.B. einer 0,5 bis 1 gew.-%igen Salzsäure, versetzt, etwa 1 h bei 90 bis 100°C nachrührt, heiß filtriert, das Produkt mit heißem Wasser bis zum neutralen Ablauf wäscht und bei 100 bis 120°C im Vakuum trocknet. Eine Rückgewinnung des organischen Lösungsmittels, wie unten beschrieben, ist bei dieser Verfahrensvariante allerdings nicht möglich. Überschüssiges Amin kann jedoch größtenteils in Form seines Hydrochlorids durch Aussalzen mit Natriumchlorid isoliert und durch Rekristallisation aus Wasser oder aliphatischen Alkoholen gereinigt werden. Nach Freisetzung des nichtumgesetzten Amins mit verdünnter wäßriger Base kann dieses wieder in die Reaktion zurückgeführt werden.

Bei der bevorzugten Ausführungsform des erfindungsgemäßen Verfahrens zur Herstellung der Naphthalimide II gewinnt man das Lösungsmittel zusammen mit nichtungesetztem Amin zurück, indem man das Reaktionsgemisch nach Abtrennung des Naphthalimids II einer Extraktion oder einer azeotropen Destillation unter Normaldruck unterwirft. Bei Verwendung eines derartigen Recyclats als Lösungsmittel kann das Molverhältnis Amin zu Naphthalin-1,8-dicarbonsäureanhydrid bis auf 1:1 erniedrigt werden. Bei der extraktiven Reinigung wird das überschüssiges Amin enthaltende Lösungsmittel zweckmäßigerweise entweder basisch extrahiert und anschließend getrocknet oder über einen Aktivkohle/Alkalimetallhydroxid-Filter (vorzugsweise wird z.B. festes Kaliumhydroxid verwendet) geleitet.

Mit Hilfe des erfindungsgemäßen Verfahrens können alle Naphthalimide II vorteilhaft auf einfache, wirtschaftliche Weise in hohen Ausbeuten (üblicherweise > 80%) hergestellt werden. Auch sterisch stark gehinderte Amine wie 2,6-Dimethylanilin, 2,6-Diisopropylanilin oder 2,6-Di-tert.-butylanilin und sehr reaktionsträge aromatische Amine wie p-Aminoazobenzol können problemlos umgesetzt werden.

Die chemische Reinheit der erfindungsgemäß hergestellten Naphthalimide II liegt in der Regel bereits bei > 97%. Falls gewünscht, kann ihre Reinheit durch weitere, für derartige Verbindungen übliche Reinigungsschritte wie Rekristallisation aus aliphatischen Carbonsäuren wie Essigsäure, N,N-disubstituierten Carbonsäureamiden wie N,N-Dimethylacetamid oder stickstoffhaltigen Heterocyclen wie N-Methyl-2-pyrrolidon sowie Halogenkohlenwasserstoffen oder Fraktionierung in Mineralsäuren wie Schwefelsäure auf > 99% erhöht werden.

### Beispiele

### A) Herstellung von Naphthalimiden II

### Beispiele 1 bis 25

Eine Mischung von 208,6 g (1 mol) Naphthalin-1,8-dicarbonsäureanhydrid (95 %ig), x mol des primären Amins III, y g Katalysator K und z ml Lösungsmittel L wurde t h unter Stickstoff auf T°C erhitzt (in Beispiel 1 unter dem sich entwickelnden Eigendruck des flüchtigen Amins).

### Verfahrensvariante V1:

Nach Abkühlen auf Raumtemperatur (und Entspannen im Fall von Beispiel 1) wurde das ausgefallene Reaktionsprodukt abgesaugt (in Beispiel 2, 3, 5, 6, 8, 11-13, 24 und 25 wurde die Ausfällung des Produkts zuvor durch einen Zusatz von etwa der gleichen Menge Methanol vervollständigt), mit dem Lösungsmittel (Beispiel 7) bzw. Methanol (restliche Beispiele) gewaschen, bis im ablaufenden Filtrat kein freies Amin mehr nachweisbar war, und bei 100°C im Vakuum getrocknet.

### Verfahrensvariante V2:

Nach Abkühlen auf etwa 95°C wurde das Reaktionsgemisch in 1 h unter Konstanthaltung der Temperatur mit 2 1 einer 0,5 gew.-%igen Salzsäure versetzt und 1 h bei dieser Temperatur nachgerührt. Das ausgefallene Reaktionsprodukt wurde heiß abfiltriert, mit heißem Wasser neutral gewaschen und bei 120°C im Vakuum getrocknet.

Weitere Einzelheiten zu diesen Versuchen sowie deren Ergebnisse sind in Tabelle 1 zusammengestellt. Dabei bedeuten:
- ZnAc:: Zinkacetatdihydrat
- HAc:: Essigsäure
- NMP:: N-Methyl-2-pyrrolidon
- DMA:: N,N-Dimethylacetamid
- DMF:: N,N-Dimethylformamid
- TSS:: p-Toluolsulfonsäure

**Tabelle 1**

| Bsp | V | III | x [mol] | Kat. K | y [g] | L | z [ml] | t [h] | T [°C] | Ausbeute [%] | Reinheit [%] | Smp. [°C] | Farbe |
|---|---|---|---|---|---|---|---|---|---|---|---|---|---|
| 1 | V1 | Methylamin | 1,5 | HAc | 100 | NMP | 400 | 4 | 50 | 95 | 98 | 207-208 | farblos |
| 2 | V1 | 5-Nonylamin | 1,2 | ZnAc | 80 | NMP | 600 | 3 | 160 | 87 | 98,5 | 73-74 | farblos |
| 3 | V1 | n-Dodecylamin | 1,05 | ZnAc | 40 | NMP | 800 | 4 | 140 | 92 | 98 | 56-58 | farblos |
| 4 | V1 | N,N-Dimethylaminopropylamin | 1,1 | ZnAc | 60 | NMP | 600 | 3 | 130 | 88 | 97 | 114-116 | farblos |
| 5 | V1 | Cyclohexylamin | 1,1 | ZnAc | 80 | NMP | 600 | 4 | 125 | 96 | 97,5 | 230-232 | blaß-gelb |
| 6 | V1 | Cyclohexylamin | 1,1 | HAc | 100 | NMP | 600 | 4 | 125 | 92 | 98 | 230-232 | blaß-gelb |
| 7 | V1 | Cyclohexylamin | 1,1 | - | - | Phenol | 900 | 2 | 140 | 90 | 97 | 229-232 | blaß-gelb |
| 8 | V1 | Cyclohexylamin | 1,1 | ZnAc | 80 | DMA | 800 | 4 | 125 | 94 | 98 | 230-232 | blaß-gelb |
| 9 | V1 | Anilin | 1,1 | ZnAc | 60 | NMP | 600 | 3 | 125 | 85 | 98,5 | 201-203 | farblos |
| 10 | V1 | 2-Methylanilin | 1,1 | ZnAc | 80 | NMP | 600 | 3 | 130 | 81 | 98 | 220-222 | farblos |
| 11 | V1 | 3,5-Dimethylanilin | 1,2 | ZnAc | 40 | NMP | 400 | 1,5 | 140 | 84 | 98 | 200-201 | farblos |
| 12 | V1 | 3,5-Dimethylanilin | 1,2 | ZnAc | 80 | Chinolin | 1000 | 1,5 | 160 | 61 | 97 | 199-201 | farblos |
| 13 | V2 | 3,5-Dimethylanilin | 1,2 | ZnAc | 30 | NMP | 400 | 2 | 150 | 99 | 97 | 199-201 | farblos |
| 14 | V1 | 2,6-Dimethylanilin | 1,1 | ZnAc | 80 | NMP | 400 | 4 | 185 | 84 | 99 | 223-225 | farblos |
| 15 | V1 | 2,6-Diisopropylanilin | 1,0 | ZnAc | 80 | DMA | 1000 | 8 | 155 | 57 | 97 | 295-297 | farblos |
| 16 | V1 | 2,6-Diisopropylanilin | 1,05 | ZnAc | 40 | NMP | 1000 | 8 | 202 | 82 | 98 | 296-298 | farblos |
| 17 | V1 | 2,6-Diisopropylanilin | 1,2 | ZnAc | 80 | Chinolin | 1000 | 4 | 230 | 88 | 98,5* | 298-299 | farblos |
| 18 | V1 | 2,6-Diisopropylanilin | 1,2 | ZnAc | 20 | DMF | 1000 | 8 | 150 | 54 | 97* | 295-297 | farblos |
| 19 | V1 | 2,6-Diisopropylanilin | 1, 1 | TSS | 50 | NMP | 850 | 8 | 202 | 91 | 98 | 297-2991 | farblos |
| 20 | V1 | 2,5-Di-tert.-butylanilin | 1,1 | ZnAc | 80 | NMP | 850 | 8 | 202 | 85 | 98,5 | 198-200 | farblos |
| 21 | V1 | p-Aminoazobenzol | 1,0 | ZnAc | 80 | NMP | 650 | 4 | 140 | 80 | 98,5 | 278-279 | orangegelb |
| 22 | V1 | p-Aminoazobenzol | 1,0 | ZnAc | 80 | DMA | 700 | 4 | 140 | 76 | 98 | 277-279 | orangegelb |
| 23 | V1 | p-Aminoazobenzol | 1,0 | ZnAc | 80 | Chinolin | 1000 | 2,5 | 160 | 79 | 98,5 | 278-279 | orangegelb |
| 24 | V1 | Benzylamin | 1,05 | ZnAc | 60 | NMP | 600 | 6 | 130 | 92 | 98 | 194-196 | farblos |
| 25 | V1 | 14-Methoxybenzylamin | 1,05 | ZnAc | 80 | NMP | 600 | 6 | 130 | 87 | 98,5 | 187-188 | farblos |

| | | | | | | | | | | | | | |
|---|---|---|---|---|---|---|---|---|---|---|---|---|---|
| *: nach Rekristallisation aus Eisessig | | | | | | | | | | | | | |

### Beispiele 26 bis 28: Rückgewinnung von Amin und Lösungsmittel

### Beispiel 26: Rein extraktive Aufreinigung

1 1 des abgetrennten Lösungsmittel/Amin-Gemisches aus Beispiel 16 wurde mehrfach mit einer 2 gew.-%igen wäßrigen Kaliumhydroxidlösung extrahiert, bis im wäßrigen Extrakt keine Naphthalsäurederivate mehr feststellbar waren, mit Wasser hydroxidfrei gewaschen und anschließend über festem Kaliumcarbonat getrocknet. Das so gewonnene Lösungsmittelrecyclat (950 ml) enthielt 18,3 g/l 2,6-Diisopropylanilin und konnte für die gleiche Umsetzung ohne Einschränkungen wieder eingesetzt werden (vgl. Beispiel 29); abhängig von der Menge an unpolaren Verunreinigungen im Naphthalsäureanhydrid bzw. von der Reinheit des eingesetzten primären Amins mußte alle 2 bis 5 Zyklen eine destillative Aufarbeitung des Lösungsmittels gemäß Beispiel 27 oder eine Aktivkohlebehandlung erfolgen.

### Beispiele 27 bis 28: Kombinierte extraktive und destillative Aufreinigung

1 1 des abgetrennten Lösungsmittel/Amin-Gemisches aus Beispiel 16 (Beispiel 27) bzw. Beispiel 21 (Beispiel 28) wurde mehrfach mit einer 2 gew.-%igen wäßrigen Kaliumhydroxidlösung extrahiert, bis im wäßrigen Extrakt keine Naphthalsäurederivate mehr feststellbar waren, und anschließend bei Normaldruck ohne Kolonne unter Stickstoff destilliert; der bis zu einer Temperatur von 180°C übergehende Vorlauf wurde verworfen. In Beispiel 27 wurden 870 ml eines farblosen N-Methyl-2-pyrrolidondestillates, in Beispiel 28 840 ml eines gelbgefärbten Chinolindestillates erhalten, welche 34,2 g 2,6-Diisopropylanilin bzw. 33,1 g p-Aminoazobenzol enthielten; die Rückgewinnungsrate an Lösungsmittel betrug in beiden Fällen 95%, die an nichtumgesetztem Amin 84% (Beispiel 27) bzw. 81% (Beispiel 28).

### Beispiel 29: Wiederholung der Umsetzung aus Beispiel 16 mit aufgereinigtem Lösungsmittel aus Beispiel 26

Die Wiederholung der Umsetzung aus Beispiel 16 mit 1 l aufgereinigtem Lösungsmittel aus Beispiel 26 und einer auf 1 mol reduzierten Menge an neuzugesetztem primärem Amin III lieferte, unter Beibehaltung aller anderen Reaktionsparameter, 296,2 g N-2',6'-Diisopropylphenylnaphthalin-1,8-dicarbonsäureimid (II) als weißes, kristallines Pulver mit einer Reinheit von 98%, was einer Ausbeute von 85% entspricht.

### B) Herstellung von Perylimiden I

### Beispiele 30 bis 83

Eine Lösung von 0,1 mol (a g) des Naphthalimids II in b₁ ml des Lösungsmittel L₁ wurde unter Rühren unter Stickstoff auf T₁°C erhitzt.

Bei dieser Temperatur wurden x molare Äquivalente ("mÄq") (y g ) der Base B entweder als Lösung (L₂) in 150 ml tert.-Butylalkohol (TBA) bzw. 100 ml tert.-Amylalkohol (TAA) so zudosiert, daß die Reaktionstemperatur nicht mehr als 5°C unter T₁°C abfiel, wobei die niedriger siedenden Hilfslösungsmittel TBA bzw. TAA kontinuierlich abdestilliert wurden, oder portionsweise als Feststoff zugegeben.

In Beispiel 32-33 wurden vor der Zugabe der Base noch 4,0 g NMP, in Beispiel 42-43 3,0 g NMP, in Beispiel 54-55 4,5 g NMP, in Beispiel 72-73 7,5 g NMP und in Beispiel 80-81 5,0 g NMP als Phasenvermittler zugesetzt.

Nach einer Nachrührzeit von t₁ h bei T₁°C und Abkühlen des Reaktionsgemisches auf 30°C (Beispiel 30-45) bzw. 60°C (Beispiel 46-83) wurde der ausgefallene, schwarzviolette Niederschlag unter Schutzgas abfiltriert, nacheinander mit 200 ml des Lösungsmittels L₁ sowie 200 ml Tetrahydrofuran (Beispiel 30-37) bzw. Petrolether (Beispiel 38-83) gewaschen und dann unter Rühren in 500 ml des Lösungsmittels L₃ eingetragen (Beispiel 30-45) bzw. mit 750 ml des heißen Lösungsmittels L₃ (Beispiel 46-83) extrahiert.

Die entstandene Suspension bzw. Lösung wurde durch portionsweise Zugabe von Eisessig oder halbkonzentrierter Schwefelsäure auf den jeweils gewünschten pH-Wert p eingestellt und dann gemäß den folgenden Verfahrensvarianten reoxidiert, wobei die Reoxidation durch einen Farbumschlag von schwarzviolett nach dunkelrot bis orangerot (je nach dem eingesetzten Substrat) bis zur Farbkonstanz zu verfolgen war:

### Verfahrensvariante VA: Reoxidation mit Wasserstoffperoxid

35 ml einer 30 gew.-%igen Wasserstoffperoxidlösung wurden zugegeben, dann wurde der Ansatz t₃ h auf T₃°C erhitzt und anschließend unter Abkühlen auf Raumtemperatur 1-2 h nachgerührt.

### Verfahrensvariante VB: Reoxidation mit Luftsauerstoff

Der Ansatz wurde auf T₃°C erhitzt, dann wurde t₃ h Luft (ca. 50-60 l/h) eingeleitet. Anschließend wurde der Ansatz unter langsamem Abkühlen auf Raumtemperatur und weiterer Luftzufuhr 3-4 h nachgerührt.

Die Aufarbeitung erfolgte bei beiden Verfahrensvarianten (in Beispiel 30, 31 und 36 nach Zugabe von 500 ml verdünnter Schwefelsäure) durch kurzes Erhitzen auf 70-80°C, Abfiltrieren des ausgefallenen Produkts und Waschen zunächst mit Wasser und dann mit Methanol bis zum neutralen Ablauf und Trocknen bei 100°C im Vakuum.

Weitere Einzelheiten zu diesen Versuchen sowie deren Ergebnisse sind in Tabelle 2 zusammengestellt. Die bei Beispiel 46-83 in Klammern angegebenen Ausbeuten sind jeweils auf den maximal möglichen Umsatz von 50% bezogen.

Dabei bedeuten:
- Exxsol:: Exxsol^{®} D 80; Gemisch aus hochsiedenden (Sdp. > 230°C) Mono- und Bicycloalkanen aus dem Rohöl-Crackprozeß (Exxon Chemicals Inc.)
- B1:: Kalium-tert.-butylat
- B2:: Kaliummethylat
- B3:: Kaliumhydroxid
- TBA:: tert.-Butylalkohol
- TAA:: tert.-Amylalkohol
- verd. HCl:: 5 gew.-%ige Salzsäure

**Tabelle 2**

| Bsp | V | II aus Bsp | a [g] | L₁ | b₁ [ml ] | T₁ [°C] | B | x/y [mÄq]/[g] | L₂ | t₁ [h] | L₃ | pH p | T₃ [°C] | t₃ [h] | Ausbeute [g]/[%] | Reinheit [%] |
|---|---|---|---|---|---|---|---|---|---|---|---|---|---|---|---|---|
| 30 | VA | 1 | 21,1 | Dekalin | 200 | 120 | B1 | 2,2/24,6 | - | 2,0 | Wasser | 10 | 40 | 4 | 19,2/92 | > 95 |
| 31 | VB | 1 | 21,1 | Xylol | 300 | 120 | B2 | 2,2/17,5 | - | 5,0 | Methanol | 9 | 60 | 2 | 18,8/90 | > 95 |
| 32 | VA | 1 | 21,1 | Exxsol | 250 | 130 | B3 | 2,5/13,9 | - | 5,0 | verd.HCl | < 1 | 40 | 4 | 18,4/88 | > 95 |
| 33 | VB | 1 | 21,1 | Xylol | 300 | 130 | B2 | 2,4/19,1 | - | 2,0 | NMP | 6-7 | 60 | 2 | 17,8/85 | > 95 |
| 34 | VA | 2 | 32,3 | Dekalin | 300 | 130 | B2 | 2,4/19,1 | - | 3,5 | Methanol | 6-7 | 40 | 4 | 26,7/83 | > 98 |
| 35 | VB | 2 | 32,3 | Dekalin | 300 | 130 | B1 | 2,1/23,5 | - | 2,0 | Eisessig | 2-3 | 50 | 3 | 24,1/75 | > 98 |
| 36 | VA | 3 | 36,6 | Dekalin | 300 | 130 | B1 | 2,1/23,5 | - | 2,0 | Methanol | 9 | 40 | 4 | 30,9/85 | > 98 |
| 37 | VB | 3 | 36,6 | Dekalin | 300 | 130 | B1 | 2,1/23,5 | - | 2,0 | Eisessig | 2-3 | 50 | 3 | 29,8/82 | > 98 |
| 38 | VA | 4 | 28,2 | Xyol | 500 | 130 | B1 | 2,2/24,6 | - | 2,0 | Wasser | 5-6 | 40 | 4 | 21,3/76 | > 95 |
| 39 | VA | 4 | 28,2 | Exxsol | 450 | 140 | B1 | 2,2/24,6 | TBA | 0,5 | Methanol | 5-6 | 40 | 4 | 21,9/78 | > 95 |
| 40 | VA | 6 | 27,9 | Mesitylen | 200 | 120 | B2 | 2,4/19,1 | - | 1,0 | verd.HCl | < 1 | 40 | 4 | 21,1/76 | > 98 |
| 41 | VB | 6 | 27,9 | Mesitylen | 200 | 120 | B2 | 2,4/19,1 | - | 1,0 | verd.HCl | < 1 | 60 | 2 | 20,5/74 | > 98 |
| 42 | VA | 6 | 27,9 | Exxsol | 250 | 120 | B2 | 2,2/17,5 | - | 0,5 | Eisessig | 2-3 | 40 | 4 | 23,6/85 | > 98 |
| 43 | VB | 6 | 27,9 | Exxsol | 250 | 120 | B2 | 2,2/17,5 | - | 0,5 | Eisessig | 2-3 | 50 | 3 | 23,0-/83 | > 98 |
| 44 | VA | 6 | 27,9 | Dekalin | 200 | 120 | B1 | 2,2/24,6 | TBA | 0,5 | Eisessig | 2-3 | 40 | 4 | 25,0/90 | > 98 |
| 45 | VB | 6 | 27,9 | Dekalin | 200 | 120 | B1 | 2,2/24,6 | TBA | 0,5 | Eisessig | 2-3 | 50 | 3 | 24,4/88 | > 98 |
| 46 | VA | 9 | 27,3 | Dekalin | 400 | 180 | B1 | 2,1/23,5 | - | 0,5 | Wasser | 3-4 | 40 | 4 | 11,7/(86) | > 95 |
| 47 | VB | 9 | 27,3 | Dekalin | 400 | 180 | B1 | 2,1/23,5 | - | 0,5 | Wasser | 3-4 | 50 | 3 | 11,4/(84) | > 95 |
| 48 | VA | 10 | 28,7 | Xylol | 400 | 160 | B1 | 2,2/24,6 | - | 2,0 | Wasser | 3-4 | 40 | 4 | 13,0/(91) | > 98 |
| 49 | VB | 10 | 28,7 | Xylol | 400 | 160 | B1 | 2,2/24,6 | - | 2,0 | Wasser | 3-4 | 50 | 3 | 12,0/(87) | > 98 |
| 50 | VA | 10 | 28,7 | Dekalin | 400 | 160 | B1 | 2,1/23,5 | TAA | 1,0 | Methanol | 3-4 | 40 | 4 | 13,3/(93) | > 98 |
| 51 | VB | 10 | 28,7 | Dekalin | 400 | 160 | B1 | 2,1/23,5 | TAA | 1,0 | Methanol | 3-4 | 50 | 3 | 12,7/(89) | > 98 |
| 52 | VA | 13 | 30,1 | Mesitylen | 500 | 180 | B1 | 2,2/24,6 | - | 1,0 | Wasser | 3-4 | 40 | 4 | 12,4/(83) | > 98 |
| 53 | VB | 13 | 30,1 | Mesitylen | 500 | 180 | B1 | 2,2/24,6 | - | 1,0 | Wasser | 3-4 | 50 | 3 | 12,1/(81) | > 98 |
| 54 | VA | 13 | 30,1 | Exxsol | 500 | 180 | B2 | 2,2/17,5 | - | 0,5 | Wasser | 3-4 | 40 | 4 | 11,7/(78) | > 95 |
| 55 | VB | 13 | 30,1 | Exxsol | 500 | 180 | B2 | 2,2/17,5 | - | 0,5 | Wasser | 3-4 | 50 | 3 | 11,5/(77) | > 95 |
| 56 | VA | 13 | 30,1 | Dekalin | 500 | 180 | B1 | 2,1/23,5 | TAA | 0,5 | Wasser | 3-4 | 40 | 4 | 13,0/(87) | > 95 |
| 57 | VB | 13 | 30,1 | Dekalin | 500 | 180 | B1 | 2,1/23,5 | TAA | 0,5 | Wasser | 3-4 | 50 | 3 | 12,6/(84) | > 95 |
| 58 | VA | 14 | 30,1 | Exxsol | 400 | 180 | B1 | 2,1/23,5 | - | 2,0 | Wasser | 3-4 | 50 | 3 | 12,4/(83) | > 98 |
| 59 | VB | 14 | 30,1 | Exxsol | 400 | 180 | B1 | 2,1/23,5 | - | 2,0 | Wasser | 3-4 | 60 | 2 | 12,6/(84) | > 98 |
| 60 | VA | 14 | 30,1 | Dekalin | 350 | 180 | B1 | 2,1/23,5 | TAA | 1,0 | Wasser | 3-4 | 50 | 3 | 13,3/(89) | > 98 |
| 61 | VB | 14 | 30,1 | Dekalin | 350 | 180 | B1 | 2,1/23,5 | TAA | 1,0 | Wasser | 3-4 | 60 | 2 | 13,2/(88) | > 98 |
| 62 | VA | 16 | 35,7 | Dekalin | 350 | 180 | B1 | 2,1/23,5 | - | 2,0 | Methanol | 3-4 | 50 | 3 | 15,3/(86) | > 98 |
| 63 | VB | 16 | 35,7 | Dekalin | 350 | 180 | B1 | 2,1/23,5 | - | 2,0 | Methanol | 3-4 | 60 | 2 | 14,8/(83) | > 98 |
| 64 | VA | 16 | 35,7 | Dekalin | 400 | 180 | B1 | 2,1/23,5 | TAA | 0,5 | Wasser | 3-4 | 50 | 3 | 16,3/(92) | > 98 |
| 65 | VB | 16 | 35,7 | Dekalin | 400 | 180 | B1 | 2,1/23,5 | TAA | 0,5 | Wasser | 3-4 | 60 | 2 | 16,0/(90) | > 98 |
| 66 | VA | 20 | 38,6 | Mesitylen | 300 | 160 | B1 | 2,2/24,6 | - | 2,0 | Methanol | 3-4 | 50 | 3 | 16,1/(84) | > 98 |
| 67 | VB | 20 | 38,6 | Mesitylen | 300 | 160 | B1 | 2,2/24,6 | - | 2,0 | Methanol | 3-4 | 60 | 2 | 16,1/(84) | > 98 |
| 68 | VA | 20 | 38,6 | Dekalin | 400 | 180 | B1 | 2,1/23,5 | TAA | 1,0 | Wasser | 3-4 | 50 | 3 | 16,7/(87) | > 98 |
| 69 | VB | 20 | 38,6 | Dekalin | 400 | 180 | B1 | 2,1/23,5 | TAA | 1,0 | Wasser | 3-4 | 60 | 2 | 16,3/(85) | > 98 |
| 70 | VA | 21 | 37,7 | Exxsol | 800 | 200 | B1 | 2,1/23,5 | - | 3,0 | Wasser | 5-6 | 30 | 6 | 14,5/(77) | > 95 |
| 71 | VB | 21 | 37,7 | Exxsol | 800 | 200 | B1 | 2,1/23,5 | - | 3,0 | Wasser | 5-6 | 30 | 6 | 14,3/(76) | > 95 |
| 72 | VA | 21 | 37,7 | Dekalin | 600 | 180 | B2 | 2,2/17,5 | - | 2,0 | Wasser | 5-6 | 30 | 6 | 15,2/(81) | > 95 |
| 73 | VB | 21 | 37,7 | Dekalin | 600 | 180 | B2 | 2,2/17,5 | - | 2,0 | Wasser | 5-6 | 30 | 6 | 14,8/(79) | > 95 |
| 74 | VA | 21 | 37,7 | Dekalin | 750 | 180 | B1 | 2,1/23,5 | TAA | 1,0 | Wasser | 5-6 | 30 | 6 | 15,4/(82) | > 95 |
| 75 | VB | 21 | 37,7 | Dekalin | 750 | 180 | B1 | 2,1/23,5 | TAA | 1,0 | Wasser | 5-6 | 30 | 6 | 15,4/(82) | > 95 |
| 76 | VA | 24 | 28,7 | Exxsol | 500 | 160 | B1 | 2,1/23,5 | - | 2,0 | Wasser | 3-4 | 40 | 4 | 11,3/(79) | > 96 |
| 77 | VB | 24 | 28,7 | Dekalin | 500 | 160 | B1 | 2,1/23,5 | - | 2,0 | Wasser | 3-4 | 60 | 2 | 11,1/(78) | > 96 |

| Bsp | V | II aus Bsp | a [g] | L₁ | b₁ [ml] | T₁ [°C] | B | x/y [mÄq]/[g] | L₂ | t₁ [h] | L₃ | pH p | T₃ [°C] | t₃ [h] | Ausbeute [g]/[%] | Reinheit [%] |
|---|---|---|---|---|---|---|---|---|---|---|---|---|---|---|---|---|
| 78 | VA | 25 | 31,7 | Mesitylen | 600 | 160 | B1 | 2,2/24,6 | - | 2,5 | Wasser | 3-4 | 40 | 4 | 11,7/(74) | > 96 |
| 79 | VB | 25 | 31,7 | Mesitylen | 600 | 160 | B1 | 2,2/24,6 | - | 2,5 | Wasser | 3-4 | 60 | 2 | 11,4/(72) | > 96 |
| 80 | VA | 25 | 31,7 | Dekalin | 500 | 180 | B2 | 2,2/17,5 | - | 1,0 | Wasser | 3-4 | 40 | 4 | 12,1/(77) | > 96 |
| 81 | VB | 25 | 31,7 | Dekalin | 500 | 180 | B2 | 2,2/17,5 | - | 1,0 | Wasser | 3-4 | 60 | 2 | 12,1/(77) | > 96 |
| 82 | VA | 25 | 31,7 | Dekalin | 600 | 180 | B1 | 2,1/23,5 | TAA | 0,5 | Wasser | 3-4 | 40 | 4 | 13,4/(85) | > 96 |
| 83 | VB | 25 | 31,7 | Dekalin | 600 | 180 | B1 | 2,1/23,5 | TAA | 0,5 | Wasser | 3-4 | 60 | 2 | 12,9/(82) | > 96 |

### Beispiele 84 bis 87: Quasikontinuierliche Fahrweise

Eine Lösung von 0,1 mol der Naphthalimide II aus den Beispielen 1 (21,1 g; Beispiel 84), 6 (27,9 g; Beispiel 85), 13 (30,1 g; Beispiel 86) und 16 (35,7 g; Beispiel 87) in jeweils 500 ml 120°C heißem Dekalin wurde kontinuierlich in einem Rohrreaktor mit Y-förmiger Mischdüse (Innendurchmesser 1 mm) bei 180°C mit einer ebenfalls 120°C heißen Lösung von 0,22 mol (24,6 g) Kalium-tert.-butylat in 400 ml Dekalin gemischt und zur Reaktion gebracht. Die Zudosierungsgeschwindigkeiten der beiden Lösungen und die Rohrdimensionen wurden so gewählt, daß sich Reaktor-Verweilzeiten von 120 s ergaben.

Das jeweils aus dem Reaktor austretende Reaktionsgemisch wurde auf 50°C abgeschreckt, gemäß Verfahrensvariante VA mit Wasserstoffperoxid reoxidiert und wie oben beschrieben aufgearbeitet.

Es wurden 19,0 g (Beispiel 84), 22,7 g (Beispiel 85), 12,0 g (Beispiel 86) bzw. 15,5 g (Beispiel 87) der entsprechenden Perylimide I erhalten, was auf den Umsatz bezogenen Ausbeuten von jeweils 91%, 82%, 80% bzw. 87% entspricht. Die Produktreinheiten lagen bei > 95% (Beispiele 84 und 86) bzw. > 98% (Beispiele 85 und 87).

### Beispiele 88 und 89: Rückgewinnung von Naphtalimid II

Die Rekristallisation des nach der Extraktion des Küpensalzes am Ende der Dimerisierung verbleibenden Filterkuchens aus Beispiel 56 (Beispiel 88) bzw. Beispiel 64 (Beispiel 89) aus N-Methyl-2-pyrrolidon lieferte 15,4 g bzw. 17,0 g des entsprechenden unumgesetzten Naphthalimids II mit einer Reinheit > 97%, was einer Rückgewinnungsrate von 90% (Beispiel 88) bzw. 88% (Beispiel 89) entspricht.

### Beispiel 90: Wiederholung der Umsetzung aus Beispiel 64 mit aufgereinigtem Naphthalimid aus Beispiel 89

Die Wiederholung der Umsetzung aus Beispiel 64 mit 35,7 g aufgereinigtem N-(2,6-Diisopropylphenyl)naphthalimid aus Beispiel 89 ergab 16,2 g N,N'-Bis(2,6-diisopropylphenyl)perylen-3,4:9,10-tetracarbonsäurediimid als orangerotes, kristallines Pulver mit einer Reinheit > 98%, was einer Ausbeute von 91% entspricht.

### B) Herstellung von Perylen-3,4:9,10-tetracarbonsäuredianhydrid Beispiel 91

Das gemäß der in Beispiel 44 beschriebenen Vorgehensweise erhaltene Küpensalz von N,N'-Dicyclohexylperylen-3,4:9,10-tetra-carbonsäurediimid wurde unter Rühren in 500 ml Toluol suspendiert, portionsweise mit insgesamt 0,4 mol (27,2 g) Natriumethylat versetzt und auf 100°C erwärmt. Bei dieser Temperatur wurden unter Rühren kontinuierlich über einen Zeitraum von 20 min insgesamt 60 ml einer 15 gew.-%igen Wasserstoffperoxidlösung zudosiert.

Nach einer Nachrührzeit von 10 min und Abkühlen auf Raumtemperatur wurde der entstandene gelbbraune Niederschlag abfiltriert, mit Isopropanol neutral gewaschen, in die 50-fache Menge an 10 gew.-%iger Salzsäure eingetragen, 10 min zum Sieden erhitzt, auf Raumtemperatur abgekühlt, abfiltriert, mit Wasser neutral gewaschen und getrocknet.

Es wurden 17,3 g Perylen-3,4:9,10-tetracarbonsäuredianhydrid als dunkelrotes amorphes Pulver mit einer Reinheit > 97% erhalten, was einer Ausbeute von 88% entspricht.

### Beispiel 92

Das gemäß der in Beispiel 56 beschriebenen Vorgehensweise erhaltene Küpensalz von N,N'-Bis(3,5-dimethylphenyl)perylen-3,4:9,10-tetracarbonsäurediimid wurde mit insgesamt 600 ml einer sauerstofffreien, 60°C warmen Mischung aus Isopropanol und Wasser im Verhältnis 4:1 aus dem Filterkuchen der Dimerisierungsreaktion extrahiert und mit 90 g Kaliumhydroxid versetzt.

Nach einer Nachrührzeit von 5 h bei 80°C und Abkühlen auf Raumtemperatur wurde der entstandene gelbbraune Niederschlag abfiltriert, mit Isopropanol neutral gewaschen, in die 50-fache Menge an 10 gew.-%iger Salzsäure eingetragen, 10 min zum Sieden erhitzt, auf Raumtemperatur abgekühlt, abfiltriert, mit Wasser neutral gewaschen und getrocknet.

Es wurden 8,2 g Perylen-3,4:9,10-tetracarbonsäuredianhydrid als dunkelrotes amorphes Pulver mit einer Reinheit > 98%, was einer Ausbeute von 84%, bezogen auf den maximal möglichen Umsatz von 50%, entspricht.

## Patentansprüche

1. Verfahren zur Herstellung von Perylen-3,4:9,10-tetracarbonsäurediimiden der allgemeinen Formel I 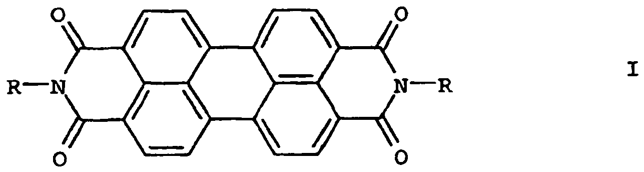 in der die Variablen folgende Bedeutung haben:
R C₁-C₃₀-Alkyl, dessen Kohlenstoffkette durch eine oder mehrere Gruppierungen -O- unterbrochen und/oder das durch C₅-C₈-Cycloalkyl, das durch C₁-C₆-Alkyl ein- oder mehrfach substituiert sein kann, Phenyl oder Phenyl-C₁-C₆-alkyl, das jeweils durch C₁-C₁₈-Alkyl und/oder C₁-C₆-Alkoxy ein-oder mehrfach substituiert sein kann, -OCOR¹, -N(R¹)₂, -SO₂NH₂, -SO₂N(R¹)₂, -CON(R¹)₂ und/oder -COOR¹ ein- oder mehrfach substituiert sein kann;
C₅-C₈-Cycloalkyl, dessen Kohlenstoffgerüst durch eine oder mehrere Gruppierungen -O-, -S- und/oder -NR²- unterbrochen sein kann und/oder das durch C₁-C₆-Alkyl ein-oder mehrfach substituiert sein kann;
Phenyl, Phenyl-C₁-C₆-alkyl, Naphthyl oder Hetaryl, das jeweils durch C₁-C₁₈-Alkyl, C₁-C₆-Alkoxy, Phenylazo, Naphthylazo, Pyridylazo, Pyrimidylazo, Cyano, -N(R¹)₂, -CON(R¹)₂ und/ oder -COOR¹ ein- oder mehrfach substituiert sein kann;
R¹ C₁-C₆-Alkyl, C₅-C₈-Cycloalkyl, Phenyl oder Phenyl-C₁-C₆-alkyl;
R² C₁-C₆-Alkyl, Phenyl oder Phenyl-C₁-C₆-alkyl,
durch Dimerisierung eines Naphthalin-1,8-dicarbonsäureimids der Formel II 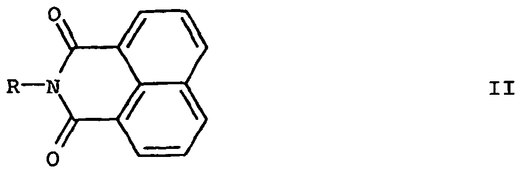 **dadurch gekennzeichnet, daß** man die Dimerisierung in einem im wesentlichen aus einem unpolar-aprotischen organischen Lösungsmittel und einer alkalimetallhaltigen Base bestehenden, weitgehend homogenen Reaktionsmedium durchführt und die dabei als Alkalimetallsalz anfallende Leukoform des Perylen-3,4:9,10-tetracarbonsäurediimids anschließend in Gegenwart eines polaren Lösungsmittels reoxidiert.

2. Verfahren zur Herstellung von Perylen-3,4:9,10-tetracarbonsäuredianhydrid, **dadurch gekennzeichnet, daß** man ein Naphthalin-1,8-dicarbonsäureimid der Formel IIa 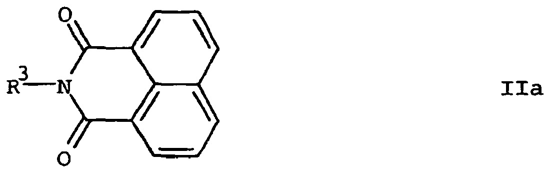 in der R³ Cyclohexyl oder Phenyl, das jeweils durch bis zu drei C₁-C₄-Alkylreste substituiert sein kann, bedeutet, in einem im wesentlichen aus einem unpolar-aprotischen organischen Lösungsmittel und einer alkalimetallhaltigen Base bestehenden, weitgehend homogenen Reaktionsmedium dimerisiert und die anschließende Reoxidation der dabei als Alkalimetallsalz anfallenden Leukoform des Perylen-3,4:9,10-tetracarbon-säurediimids der Formel Ia 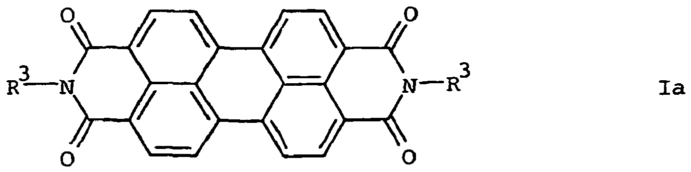 in Gegenwart eines inerten organischen Lösungsmittels, einer alkalimetallhaltigen Base und von Wasser vornimmt, so daß das Diimid zum Tetraalkalimetallsalz der Perylen-3,4:9,10-tetracarbonsäure verseift wird, und abschließend dieses Salz unter Einwirkung einer wäßrigen anorganischen Säure in das Perylen-3,4:9,10-tetracarbonsäuredianhydrid überführt.

3. Verfahren zur Herstellung von Naphthalin-1,8-dicarbonsäure-imiden der allgemeinen Formel II 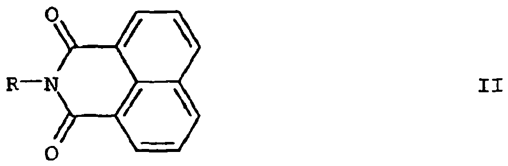 in der die Variablen folgende Bedeutung haben:
R C₁-C₃₀-Alkyl, dessen Kohlenstoffkette durch eine oder mehrere Gruppierungen -O- unterbrochen und/oder das durch C₅-C₈-Cycloalkyl, das durch C₁-C₆-Alkyl ein- oder mehrfach substituiert sein kann, Phenyl oder Phenyl-C₁-C₆-alkyl, das jeweils durch C₁-C₁₈-Alkyl und/oder C₁-C₆-Alkoxy einoder mehrfach substituiert sein kann, -OCOR¹, -N(R¹)₂, -SO₂NH₂, -SO₂N(R¹)₂, -CON(R¹)₂ und/oder -COOR¹ ein- oder mehrfach substituiert sein kann;
C₅-C₈-Cycloalkyl, dessen Kohlenstoffgerüst durch eine oder mehrere Gruppierungen -O-, -S- und/oder -NR²- unterbrochen sein kann und/oder das durch C₁-C₆-Alkyl ein-oder mehrfach substituiert sein kann;
Phenyl, Phenyl-C₁-C₆-alkyl, Naphthyl oder Hetaryl, das jeweils durch C₁-C₁₈-Alkyl, C₁-C₆-Alkoxy, Phenylazo, Naphthylazo, Pyridylazo, Pyrimidylazo, Cyano, -N(R¹)₂, -CON(R¹)₂ und/ oder -COOR¹ ein- oder mehrfach substituiert sein kann;
R¹ C₁-C₆-Alkyl, C₅-C₈-Cycloalkyl, Phenyl oder Phenyl-C₁-C₆-alkyl;
R² C₁-C₆-Alkyl, Phenyl oder Phenyl-C₁-C₆-alkyl, durch Umsetzung von Naphthalin-1,8-dicarbonsäureanhydrid mit einem primären Amin der allgemeinen Formel III
R-NH₂ III
**dadurch gekennzeichnet, daß** man die Umsetzung in Gegenwart eines polar-aprotischen organischen Lösungsmittels sowie einer organischen oder anorganischen Säure oder eines sauren Übergangsmetallsalzes als Katalysator oder in Gegenwart von Phenol vornimmt.

4. Verfahren nach Anspruch 3, **dadurch gekennzeichnet, daß** man das Reaktionsgemisch nach Abtrennung des Naphthalin-1,8-dicarbonsäureimids II und seiner Hydrolyseprodukte einer Extraktion oder einer azeotropen Destillation unter Normaldruck unterwirft und auf diese Weise das organische Lösungsmittel zusammen mit nichtumgesetztem Amin für weitere Umsetzungen zurückgewinnt.

5. Naphthalin-1,8-dicarbonsäureimide der allgemeinen Formel IIb 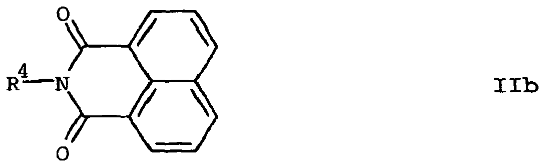 in der die Variablen folgende Bedeutung haben:
R⁴ C₁-C₃₀-Alkyl, das durch C₅-C₈-Cycloalkyl, das durch C₁-C₆-Alkyl ein- oder mehrfach substituiert sein kann, Phenyl oder Phenyl-C₁-C₆-alkyl, das jeweils durch C₁-C₁₈-Alkyl und/oder C₁-C₆-Alkoxy ein- oder mehrfach substituiert sein kann, -OCOR¹, -SO₂NH₂, -SO₂N(R¹)₂, -CON(R¹)₂ und/oder -COOR¹ ein- oder mehrfach substituiert ist und dessen Kohlenstoffkette durch eine oder mehrere Gruppierungen -O- unterbrochen sein kann;
C₅-C₈-Cycloalkyl, dessen Kohlenstoffgerüst durch eine oder mehrere Gruppierungen -O-, -S- und/oder -NR²- unterbrochen und/oder das durch C₁-C₆-Alkyl ein- oder mehrfach substituiert ist;
Phenyl oder Phenyl-C₁-C₆-alkyl, das jeweils durch C₁-C₁₈-Alkyl, C₁-C₆-Alkoxy, Phenylazo, Naphthylazo, Pyridylazo, Pyrimidylazo, Cyano, -CON(R¹)₂ und/ oder -COOR¹ ein- oder mehrfach substituiert ist;
Naphthyl, 2- oder 3-Pyrryl, 2-, 3- oder 4-Pyridyl, 2-, 4- oder 5-Pyrimidyl, 3-, 4- oder 5-Pyrazolyl, 6-Chinaldyl, 3-, 5-, 6- oder 8-Chinolinyl, 2-Benzoxazolyl, 5-Benzothiadiazolyl, oder 1- oder 5-Isochinolyl, das jeweils durch C₁-C₁₈-Alkyl, C₁-C₆-Alkoxy, Phenylazo, Naphthylazo, Pyridylazo, Pyrimidylazo, Cyano, -CON(R¹)₂ und/ oder -COOR¹ ein- oder mehrfach substituiert sein kann;
R¹ C₁-C₆-Alkyl, C₅-C₈-Cycloalkyl, Phenyl oder Phenyl-C₁-C₆-alkyl;
R² C₁-C₆-Alkyl, Phenyl oder Phenyl-C₁-C₆-alkyl.

## Claims

1. A process for preparing perylene-3,4:9,10-tetracarboxylic diimides of the general formula I 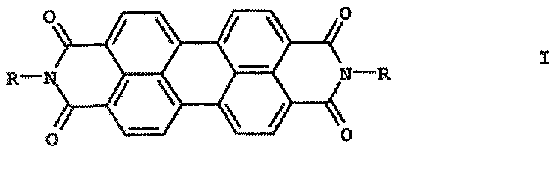 where
R is C₁-C₃₀-alkyl whose carbon chain may be interrupted by one or more -O- moieties and/or which may be substituted by one or more substituents selected from the group consisting of C₅-C₈-cycloalkyl (which may be substituted by one or more C₁-C₆-alkyl substituents), phenyl or phenyl-C₁-C₆-alkyl (which may each be substituted by one or more C₁-C₁₈-alkyl and/or C₁-C₆-alkoxy substituents), -OCOR¹, -N(R¹)₂, - SO₂NH₂, -SO₂N(R¹)₂, -CON(R¹)₂ and -COOR¹;
C₅-C₈-cycloalkyl whose carbon skeleton may be interrupted by one or more moieties selected from the group consisting of -O-, -S- and -NR²-and/or which may be substituted by one or more C₁-C₆-alkyl substituents;
phenyl, phenyl-C₁-C₆-alkyl, naphthyl or hetaryl, which may each be substituted by one or more substituents selected from the group consisting of C₁-C₁₈-alkyl, C₁-C₆-alkoxy, phenylazo, naphthylazo, pyridylazo, pyrimidylazo, cyano, - N(R¹)₂, -CON(R¹)₂ and -COOR¹;
R¹ is C₁-C₆-alkyl, C₅-C₈-cycloalkyl, phenyl or phenyl-C₁-C₆-alkyl;
R² is C₁-C₆-alkyl, phenyl or phenyl-C₁-C₆-alkyl,
by dimerizing a naphthalene-1,8-dicarboximide of the formula II 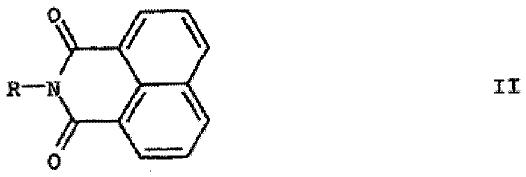 which comprises effecting said dimerizing in a largely homogeneous reaction medium consisting essentially of an apolar aprotic organic solvent and an alkali metal base and subsequently reoxidizing the resulting alkali metal salt of the leuco form of the perylene-3,4:9,10-tetracarboxylic diimide in the presence of a polar solvent.

2. A process for preparing perylene-3,4:9,10-tetracarboxylic dianhydride, which comprises dimerizing a naphthalene-1,8-dicarboximide of the formula IIa 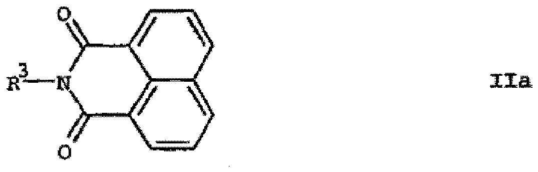 where R³ is cyclohexyl or phenyl which may each be substituted by up to three C₁-C₄-alkyl radicals, in a largely homogeneous reaction medium consisting essentially of an apolar aprotic organic solvent and an alkali metal base and effecting the subsequent reoxidation of the resulting alkali metal salt of the leuco form of the perylene-3,4:9,10-tetracarboxylic diimide of the formula Ia 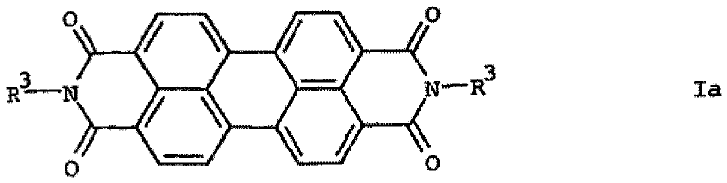 in the presence of an inert organic solvent, of an alkali metal base and of water to hydrolyze the diimide to the tetraalkali metal salt of perylene-3,4:9,10-tetracarboxylic acid and finally subjecting this to the action of an aqueous inorganic acid to convert it into perylene-3,4:9,10-tetracarboxylic dianhydride.

3. A process for preparing naphthalene-1,8-dicarboximides of the general formula II 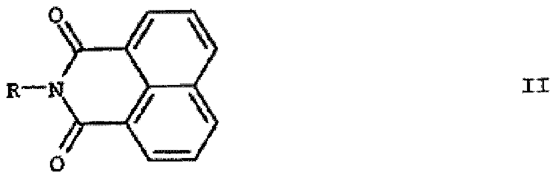 where
R is C₁-C₃₀-alkyl whose carbon chain may be interrupted by one or more -O- moieties and/or which may be substituted by one or more substituents selected from the group consisting of C₅-C₈-cycloalkyl (which may be substituted by one or more C₁-C₆-alkyl substituents), phenyl or phenyl-C₁-C₆-alkyl (which may each be substituted by one or more C₁-C₁₈-alkyl and/or C₁-C₆-alkoxy substituents), -OCOR¹, -N(R¹)₂, - SO₂NH₂, -SO₂N (R¹)₂, -CON (R¹) ₂ and -COOR¹;
C₅-C₈-cycloalkyl whose carbon scaffold may be interrupted by one or more moieties selected from the group consisting of -O-, -S- and -NR²-and/or which may be substituted by one or more C₁-C₆-alkyl substituents;
phenyl, phenyl-C₁-C₆-alkyl, naphthyl or hetaryl, which may each be substituted by one or more substituents selected from the group consisting of C₁-C₁₈-alkyl, C₁-C₆-alkoxy, phenylazo, naphthylazo, pyridylazo, pyrimidylazo, cyano, - N(R¹)₂, -CON(R¹)₂ and -COOR¹;
R¹ is C₁-C₆-alkyl, C₅-C₈-cycloalkyl, phenyl or phenyl-C₁-C₆-alkyl;
R² is C₁-C₆-alkyl, phenyl or phenyl-C₁-C₆-alkyl,
by reacting naphthalene-1,8-dicarboxylic anhydride with a primary amine of the general formula III
R-NH₂ III
which comprises effecting said reacting in the presence of a polar aprotic organic solvent and also of an organic or inorganic acid or of an acidic transition metal salt catalyst or in the presence of phenol.

4. The process according to claim 3, wherein the reaction mixture (after the naphthalene-1,8-dicarboximide II and its hydrolysis products have been removed) is subjected to an extraction or an azeotropic distillation under atmospheric pressure to recover the organic solvent together with unconverted amine for further reactions.

5. Naphthalene-1,8-dicarboximides of the general formula IIb 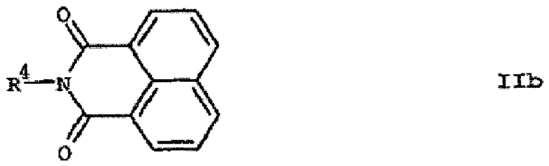 where:
R⁴ is C₁-C₃₀-alkyl which is substituted by one or more substituents selected from the group consisting of C₅-C₈-cycloalkyl (which may be substituted by one or more C₁-C₆-alkyl substituents), phenyl or phenyl-C₁-C₆-alkyl (which may each be substituted by one or more C₁-C₁₈-alkyl and/or C₁-C₆-alkoxy substituents), - OCOR¹, -SO₂NH₂, -SO₂N(R¹)₂, -CON(R¹)₂ and -COOR¹ and whose carbon chain may be interrupted by one or more -O- moieties;
C₅-C₈-cycloalkyl whose carbon skeleton is interrupted by one or more moieties selected from the group consisting of -O-, -S- and -NR²-and/or is substituted by one or more C₁-C₆-alkyl substituents;
phenyl or phenyl-C₁-C₆-alkyl which are each substituted by one or more substituents selected from the group consisting of C₁-C₁₈-alkyl, C₁-C₆-alkoxy, phenylazo, naphthylazo, pyridylazo, pyrimidylazo, cyano, -CON(R¹)₂ and - COOR¹;
naphthyl, 2- or 3-pyrryl, 2-, 3- or 4-pyridyl, 2-, 4- or 5-pyrimidyl, 3-, 4- or 5-pyrazolyl, 6-quinaldyl, 3-, 5-, 6- or 8-quinolinyl, 2-benzoxazolyl, 5-benzothiadiazolyl, or 1- or 5-isoquinolyl, which may each be substituted by one or more substituents selected from the group consisting of C₁-C₁₈-alkyl, C₁-C₆-alkoxy, phenylazo, naphthylazo, pyridylazo, pyrimidylazo, cyano, -CON(R¹)₂ and -COOR¹;
R¹ is C₁-C₆-alkyl, C₅-C₈-cycloalkyl, phenyl or phenyl-C₁-C₆-alkyl;
R² is C₁-C₆-alkyl, phenyl or phenyl-C₁-C₆-alkyl.

## Revendications

1. Procédé pour la préparation de diimides de l'acide pérylène-3,4:9,10-tétracarboxylique de formule générale I 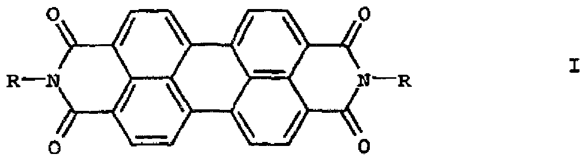 dans laquelle les variables ont la signification suivante :
R C₁-C₃₀-alkyle, dont la chaîne carbone peut être interrompue par un ou plusieurs groupements -O-et/ou qui peut être monosubstitué ou polysubstitué par C₅-C₈-cycloalkyle, qui peut être monosubstitué ou polysubstitué par C₁-C₆-alkyle ; phényle ou phënyl-C₁-C₆-alkyle, qui peut à chaque fois être monosubstitué ou polysubstitué par C₁-C₁₈-alkyle et/ou C₁-C₆-alcoxy; -OCOR¹, -N(R¹)₂, -SO₂NH₂, -SO₂N(R¹)₂, -CON(R¹)₂ et/ou -COOR¹;
C₅-C₈-cycloalkyle, dont la structure carbonée peut être interrompue par un ou plusieurs groupements -O-, -S- et/ou -NR²- et/ou qui peut être monosubstitué ou polysubstitué par C₁-C₆-alkyle ;
phényle, phényl-C₁-C₆-alkyle, naphtyle ou hétaryle, qui peut à chaque fois être monosubstitué ou polysubstitué par C₁-C₁₈-alkyle, C₁-C₆-alcoxy, phénylazo, naphtylazo, pyridylazo, pyrimidylazo, cyano, -N(R¹)₂, -CON(R¹)₂ et/ou -COOR¹ ;
R¹ C₁-C₆-alkyle, C₅-C₈-cycloalkyle, phényle ou phényl-C₁-C₆-alkyle ;
R² C₁-C₆-alkyle, phényle ou phényl-C₁-C₆-alkyle,
par dimérisation d'un imide de l'acide naphtalène-1,8-dicarboxylique de formule II 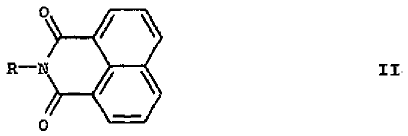 **caractérisé en ce qu'**on réalise la dimérisation dans un milieu réactionnel dans une large mesure homogène, essentiellement constitué par un solvant organique non polaire-aprotique et une base contenant un métal alcalin et la leucoforme obtenue sous forme de sel de métal alcalin du diimide de l'acide pérylène-3,4:9,10-tétracarboxylique est ensuite réoxydée en présence d'un solvant polaire.

2. Procédé pour la préparation de dianhydride de l'acide pérylène-3,4:9,10-tétracarboxylique, **caractérisé en ce qu'**on dimérise un imide de l'acide naphtalène-1,8-dicarboxylique de formule IIa 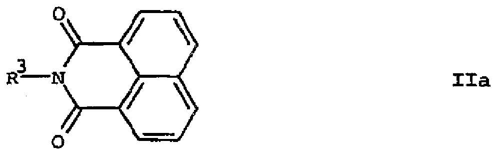 dans laquelle R³ signifie cyclohexyle ou phényle, qui peut être substitué à chaque fois par jusqu'à trois radicaux C₁-C₄-alkyle,
dans un milieu réactionnel dans une large mesure homogène, essentiellement constitué par un solvant organique non polaire-aprotique et une base contenant un métal alcalin et on réalise la réoxydation consécutive de la leucoforme obtenue sous forme de sel de métal alcalin du diimide de l'acide pérylène-3,4:9,10-tétracarboxylique de formule Ia 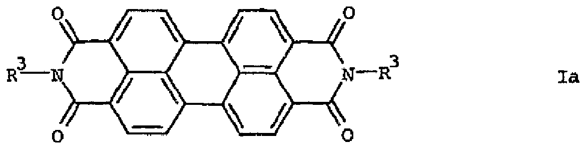 en présence d'un solvant organique inerte, d'une base contenant un métal alcalin et de l'eau, de manière telle que le diimide est saponifié en sel de tétra(métal alcalin) de l'acide pérylène-3,4:9,10-tétracarboxylique et on transforme ensuite ce sel sous l'effet d'un acide inorganique aqueux en dianhydride de l'acide pérylène-3,4:9,10-tétracarboxylique.

3. Procédé pour la préparation d'imides de l'acide naphtalène-1,8-dicarboxylique de formule générale II, 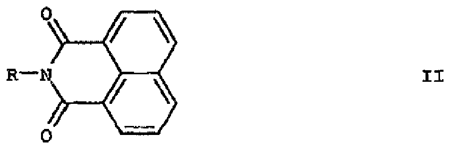 dans laquelle les variables ont la signification suivante :
R C₁-C₃₀-alkyle, dont la chaîne carbone peut être interrompue par un ou plusieurs groupements -O-et/ou qui peut être monosubstitué ou polysubstitué par C₅-C₈-cycloalkyle, qui peut être monosubstitué ou polysubstitué par C₁-C₆-alkyle ; phényle ou phényl-C₁-C₆-alkyle, qui peut à chaque fois être monosubstitué ou polysubstitué par C₁-C₁₈-alkyle et/ou C₁-C₆-alcoxy ; -OCOR¹, -N(R¹)₂, -SO₂NH₂, -SO₂N(R¹)₂, -CON(R¹)₂ et/ou -COOR¹ ;
C₅-C₈-cycloalkyle, dont la structure carbonée peut être interrompue par un ou plusieurs groupements -O-, -S- et/ou -NR²- et/ou qui peut être monosubstitué ou polysubstitué par C₁-C₆-alkyle ;
phényle, phényl-C₁-C₆-alkyle, naphtyle ou hétaryle, qui peut à chaque fois être monosubstitué ou polysubstitué par C₁-C₁₈-alkyle, C₁-C₆-alcoxy, phénylazo, naphtylazo, pyridylazo, pyrimidylazo, cyano, -N(R¹)₂, -CON(R¹)₂ et/ou -COOR¹ ;
R¹ C₁-C₆-alkyle, C₅-C₈-cycloalkyle, phényle ou phényl-C₁-C₆-alkyle ;
R² C₁-C₆-alkyle, phényle ou phényl-C₁-C₆-alkyle,
par transformation d'anhydride de l'acide naphtalène-1,8-dicarboxylique avec une amine primaire de formule générale III
R-NH₂ III
**caractérisé en ce qu'**on réalise la transformation en présence d'un solvant organique polaire-aprotique ainsi que d'un acide organique ou inorganique ou d'un sel acide de métal de transition comme catalyseur ou en présence de phénol.

4. Procédé selon la revendication 3, **caractérisé en ce qu'**on soumet le mélange réactionnel après séparation de l'imide de l'acide naphtalène-1,8-dicarboxylique II et ses produits d'hydrolyse à une extraction ou une distillation azéotropique sous pression normale et on récupère de cette manière le solvant organique ensemble avec une amine non transformée pour d'autres transformations.

5. Imides de l'acide naphtalène-1,8-dicarboxylique de formule générale IIb, 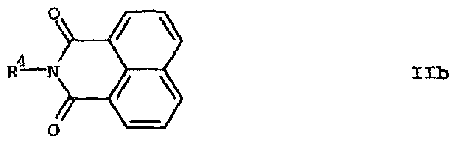 dans laquelle les variables ont la signification suivante :
R⁴ C₁-C₃₀-alkyle, qui est monosubstitué ou polysubstitué par C₅-C₈-cycloalkyle, qui peut être monosubstitué ou polysubstitué par C₁-C₆-alkyle ; phényle ou phényl-C₁-C₆-alkyle, qui peut à chaque fois être monosubstitué ou polysubstitué par C₁-C₁₈-alkyle et/ou C₁-C₆-alcoxy ; -OCOR¹, -SO₂NH₂, -SO₂N(R¹)₂, -CON(R¹)₂ et/ou -COOR¹ et dont la chaîne carbonée peut être interrompue par un ou plusieurs groupements -O- ;
C₅-C₈-cycloalkyle, dont la structure carbonée peut être interrompue par un ou plusieurs groupements -O-, -S- et/ou -NR²- et/ou qui peut être monosubstitué ou polysubstitué par C₁-C₆-alkyle ;
phényle et phényl-C₁-C₆-alkyle, qui peut à chaque fois être monosubstitué ou polysubstitué par C₁-C₁₈-alkyle, C₁-C₆-alcoxy, phénylazo, naphtylazo, pyridylazo, pyrimidylazo, cyano, -CON(R¹)₂ et/ou -COOR¹;
naphtyle, 2-pyrryle ou 3-pyrryle, 2-pyridyle, 3-pyridyle ou 4-pyridyle, 2-pyrimidyle, 4-pyrimidyle ou 5-pyrimidyle, 3-pyrazolyle, 4-pyrazolyle ou 5-pyrazolyle, 6-quinaldyle, 3-quinoléinyle, 5-quinoléinyle, 6-quinoléinyle ou 8-quinoléinyle, 2-benzoxazolyle, 5-benzothiadiazolyle, ou 1-isoquinoléyle ou 5-isoquinoléyle, qui peut être à chaque fois monosubstitué ou polysubstitué par C₁-C₁₈-alkyle, C₁-C₆-alcoxy, phénylazo, naphtylazo, pyridylazo, pyrimidylazo, cyano, -CON(R¹)₂ et/ou -COOR¹ ;
R¹ C₁-C₆-alkyle, C₅-C₈-cycloalkyle, phényle ou phényl-C₁-C₆-alkyle ;
R² C₁-C₆-alkyle, phényle ou phényl-C₁-C₆-alkyle.
